# FASCICULE DE BREVET EUROPEEN

(11) **EP 2 313 376 B1**
(45) Date de publication et mention de la délivrance du brevet: **24.12.2014**
(21) Numéro de dépôt: 09784493.0
(22) Date de dépôt: 09.07.2009
(51) Int. Cl.: C07D 237/28, C07D 405/04, C07D 409/04, A61K 31/502, A61P 25/00, A61P 35/00, C07D 237/36

(54) **DERIVES DE 1-BENZYL-CINNOLIN-4-(1H)-ONE SUBSTITUES, LEUR PREPARATION ET LEUR APPLICATION EN THERAPEUTIQUE**
SUBSTITUIERTE 1-BENZYLCINNOLIN-4(1H)-ON DERIVATE, ZUBEREITUNG DAVON UND THERAPEUTISCHE ANWENDUNG DAVON
SUBSTITUTED 1-BENZYL-CINNOLIN-4(1H)-ONE DERIVATIVES, PREPARATION THEREOF, AND THERAPEUTIC USE THEREOF

(30) Priorité: 11.07.2008 FR 0803974
(43) Date de publication de la demande: 27.04.2011
(73) Titulaire: SANOFI, 75008 Paris (FR)
(72) Inventeur: BARBAGALLO, Elodie, F-75013 Paris (FR); RINALDI-CARMONA, Murielle, F-75013 Paris (FR); ROUX, Pascale, F-75013 Paris (FR); VERNHET, Claude, F-75013 Paris (FR)
(74) Mandataire: Werner, Alain Henri
(86) Numéro de dépôt international: PCT/FR2009/051362
(87) Numéro de publication internationale: WO 2010/004215

(56) Documents cités:
- WO-A-02/42269
- E. STERN ET AL.: "Pharmacomodulations around the 4-oxo-1,4-dihydroquinoline-3-carboxamides, a class of potent CB2-selective cannabinoid receptor ligands: consequences in receptor affinity and functionality." JOURNAL OF MEDICINAL CHEMISTRY, vol. 50, 2007, pages 5471-5484, XP002515712 cité dans la demande

## Description

La présente invention a pour objet de nouveaux dérivés de 1-benzyl-cinnolin-4-(1H)-one substitués, ayant une affinité pour les récepteurs CB2 des cannabinoïdes, à leur préparation et à leur application en thérapeutique.

Le Δ⁹-THC est le principal constituant actif extrait de *Cannabis sativa* [Paton, Annual Review in Pharmacology (1975) 15 : 191-220]

De nombreux articles ont décrit non seulement des effets psychotropes des cannabinoïdes, mais aussi une influence de ces derniers sur la fonction immunitaire [Klein et al., Immunology Today (1998) 19: 373-381], le contrôle de la douleur [Pertwee, Progress in Neurobiology (2001) 63 : 569-611], de la prise alimentaire [Cota et al. International Journal of Obesity (2003) 27 : 289-301] et de nombreuses autres fonctions biologiques [Nahas et al. Marihuana and medicine (1999) Humana Press : Totowa, New Jersey, USA].

Les effets des cannabinoïdes sont dus à une interaction avec des récepteurs spécifiques de haute affinité, couplés aux protéines G, présents au niveau central et périphérique [Howlett et al., Pharmacological Reviews (2002) 54 : 161-2002].

Les effets centraux des cannabinoïdes relèvent d'un premier type de récepteur des cannabinoïdes (CB₁) présent principalement dans le cerveau mais aussi à la périphérie [Matsuda et al., Nature (1990) 346: 561-564]. Par ailleurs, Munro et al. [Nature (1993) 365 : 61-65] ont cloné un second type de récepteur aux cannabinoïdes appelé CB₂ qui est présent à la périphérie et en particulier dans les cellules du système immunitaire. Dans certaines conditions pathologiques, on observe la présence des récepteurs CB2 au niveau du cerveau.

Certains dérivés indoliques ont été cités dans l'art antérieur comme présentant une affinité pour les récepteurs CB₂ : on peut citer les demandes de brevets US 5 532 237, EP 833 818, US 4 581 354, WO 2002/42269, WO 2003/097597.

Des dérivés cinnoline ont été décrits par E. Stern et al. dans J. Med. Chem, 2007, 50, 5471-5484, en particulier le composé de formule:

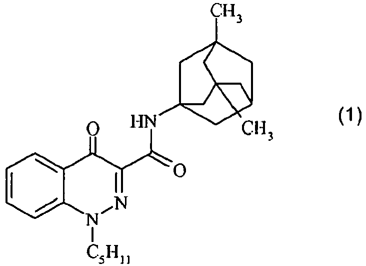

On a maintenant trouvé de nouveaux composés, dérivés de 1-benzyl-cinnolin-4-(1*H*)-one substitués, qui présentent une forte affinité et une grande sélectivité pour les récepteurs CB₂ des cannabinoïdes. Ces composés présentent un effet modulateur sur l'activité des récepteurs CB₂. Par effet modulateur on entend notamment des effets agonistes, antagonistes et/ou agonistes inverses.

La présente invention a pour objet des composés répondant à la formule (I) : 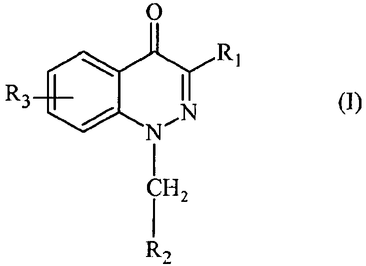 dans laquelle :
- R₁ représente :
   - un phényle non substitué ou substitué une ou plusieurs fois par des substituants choisis indépendamment parmi un atome d'halogène, un groupe Alk, un groupe OAlk, un cyano, un groupe -NHSO₂Alk, un groupe -SO₂Alk ;
   - un naphtyle non substitué ou substitué une ou plusieurs fois par des substituants choisis indépendamment parmi un atome d'halogène, un groupe Alk, un groupe OAlk;
   - un pyridyle non substitué ou substitué une ou plusieurs fois par des substituants choisis indépendamment parmi un atome d'halogène, un groupe Alk, un groupe OAlk ;
   - un 1-benzothiényle non substitué ou substitué une ou plusieurs fois par des substituants choisis indépendamment parmi un atome d'halogène, un groupe Alk, un groupe OAlk ;
   - un 1,3-benzodioxolyle non substitué ou substitué une ou plusieurs fois par des substituants choisis indépendamment parmi un atome d'halogène, un groupe Alk, un groupe OAlk ;
- R₂ représente :
   - un groupe aromatique ou hétéro-aromatique, ledit groupe étant non substitué ou substitué une ou plusieurs fois par des substituants choisis indépendamment parmi un atome d'halogène, un groupe Alk, un groupe OAlk, un cyano ;
- R₃ représente un atome d'hydrogène, un atome d'halogène, un groupe Alk, un groupe OAlk ;
- Alk représente un (C₁-C₄)alkyle non substitué ou substitué une ou plusieurs fois par un atome de fluor.

Les composés de formule (I) peuvent comporter un ou plusieurs atomes de carbone asymétriques. Ils peuvent donc exister sous forme d'énantiomères ou de diastéréoisomères. Ces énantiomères, diastéréoisomères ainsi que leurs mélanges, y compris les mélanges racémiques, font partie de l'invention.

Les composés de formule (I) peuvent exister à l'état de bases ou de sels d'addition à des acides. De tels sels d'addition font partie de l'invention.

Ces sels sont avantageusement préparés avec des acides pharmaceutiquement acceptables mais les sels d'autres acides utiles pour la purification ou l'isolement des composés de formule (I) font également partie de l'invention.

Par (C₁-C₄)alkyle, on entend un radical carboné linéaire ou ramifié de 1 à 4 atomes de carbone, tel que en particulier : méthyle, éthyle, propyle, isopropyle, butyle, isobutyle, tert-butyle.

Par (C₁-C₄)alcoxy, on entend un atome d'oxygène lié à un radical carboné linéaire ou ramifié de 1 à 4 atomes de carbone tel que le radical méthoxy, éthoxy, propoxy, isopropoxy, butoxy, sec-butoxy, tert-butoxy.

Par atome d'halogène, on entend un atome de fluor, de chlore, de brome ou d'iode.

Par groupe aromatique ou hétéro-aromatique on entend par exemple un phényle, naphtyle, pyridyle, thiadiazolyle, indolyle, indazolyle, imidazolyle, benzimidazolyle, bcnzotriazolyle, benzofurannyle, benzothiényle, benzothiazolyle, benzisothiazolyle, quinolyle, isoquinolyle, benzoxazolyle, benzisoxazolyle, benzoxazinyle, benzodioxinyle, isoxazolyle, benzopyrannyle, thiazolyle, thiényle, furyle, pyrannyle, chroményle, isobenzofurannyle, pyrrolyle, pyrazolyle, pyrazinyle, pyrimidinyle, pyridazinyle, indolizinyle, phtalazinyle, quinazolinyle, acridinyle, isothiazolyle, isochromannyle, chromannyle.

Selon la présente invention, on préfère les composés de formule (I) dans laquelle :
- R₁ représente :
   - un phényle non substitué ou substitué une ou deux fois par des substituants choisis indépendamment parmi un atome d'halogène, un groupe Alk, un groupe OAlk, un groupe -SO₂Alk ;
   - un naphtyle ;
   - un pyridyle substitué par un groupe Alk ou un groupe OAlk ;
   - un 1-benzothiényle ;
   - un 1,3-benzodioxolyle substitué par un ou plusieurs atomes de fluor ;
- R₂ représente :
   - un phényle non substitué ou substitué une ou deux fois par des substituants choisis indépendamment parmi un atome d'halogène, un groupe Alk, un cyano ;
   - un thiényle ou un pyridyle substitué par un atome d'halogène ou un groupe Alk ;
- R₃ représente un atome d'hydrogène, d'halogène ou un groupe OAlk ;
- Alk représente un (C₁-C₄)alkyle non substitué ou substitué une ou plusieurs par un atome de fluor ;
   à l'état de base ou de sel d'addition à un acide.

Particulièrement, on préfère les composés de formule (I) dans laquelle :
- R₁ représente :
   - un phényle, un 4-chlorophényle, un 2-fluorophényle, un 3-fluorophényle, un 2-méthylphényle, un 4-méthylphényle, un 3-isopropylphényle, un 2-trifluorométhylphényle, un 3-trifluorométhylphényle, un 2-méthoxyphényle, un 4-méthoxyphényle, un 2-trifluorométhoxyphényle, un 3-trifluorométhoxyphényle, un 4-trifluorométhoxyphényle, un 2,3-dichlorophényle, un 3,5-dichlorophényle, un 2,3-difluorophényle, un 2,5-difluorophényle, un 3-chloro-2-fluorophényle, un 2-chloro-3-trifluorométhylphényle, un 3-chloro-5-trifluorométhylphényle, un 4-chloro-2-trifluorométhylphényle, un 3-bromo-2-fluorophényle, un 2-fluoro-3-méthylphényle, un 2-fluoro-4-méthylphényle, un 2-fluoro-3-trifluorométhylphényle, un 3-fluoro-2-trifluorométhylphényle, un 3-fluoro-5-trifluorométhylphényle, un 3-fluoro-2-méthoxyphényle, un 4-fluoro-2-méthoxyphényle, un 2-fluoro-5-trifluorométhoxyphényle, un 3-isopropyl-6-méthoxyphényle, un 3-*tert*-butyl-6-méthoxyphényle, un 2-(méthylsulfonyl)phényle ;
   - un naphtyle ;
   - un 2-méthoxypyrid-5-yle, un 2-trifluorométhylpyrid-3-yle ;
   - un benzothièn-2-yle, un benzothièn-7-yle ;
   - un 2,2-difluorobenzodioxol-4-yle;
- R₂ représente:
   - un phényle, un 4-chlorophényle, un 2-fluorophényle, un 4-fluorophényle, un 2,4-dichlorophényle, un 2,4-difluorophényle, un 2-chloro-4-fluorophényle, un 4-chloro-2-fluorophényle, un 2-fluoro-4-méthylphényle, un 2-fluoro-4-trifluorométhylphényle, un 2-fluoro-4-cyanophényle ;
   - un 2-chlorothièn-5-yle, un 3-trifluorométhylpyrid-6-yle, un 5-chloropyrid-2-yle, un 3,5-difluoropyrid-2-yle ;
- R₃ représente un atome d'hydrogène, un atome de chlore ou un méthoxy ;
   à l'état de base ou de sel d'addition à un acide.

Parmi les composés de formule (I), objets de l'invention, on peut notamment citer les composés suivants :
- 1-(2,4-Difluorobenzyl)-3-[2-fluoro-3-(trifluorométhyl)phényl]-8-méthoxycinnolin-4(*H*)-one ;
- 3-[2-Chloro-3-(trifluorométhyl)phényl]-1-(2,4-difluorobenzyl)-8-méthoxycinnolin-4(1*H*)-one ;
- 3-[2-Chloro-3-(trifluorométhyl)phényl]-1-(2,4-difluorobenzyl)cinnolin-4(*H*)-one ;
- 3-(2,3-Dichlorophényl)-1-(2,4-difluorobenzyl)-8-méthoxycinnolin-4(1*H*)-one ;
- 1-(2,4-Difluorobenzyl)-3-[2-fluoro-3-(trifluorométhyl)phényl]cinnolin-4(1*H*)-one ;
- 1-(2,4-Difluorobenzyl)-3-[3-fluoro-2-(trifluorométhyl)phényl]cinnolin-4(1*H*)-one ;
- 1-(4-Chloro-2-fluorobenzyl)-3-[2-fluoro-3-(trifluorométhyl)phényl]cinnolin-4(1*H*)-one ;
- 1-(2,4-Difluorobenzyl)-3-(3-isopropylphényl)cinnolin-4(1*H*)-one ;
- 1-(2,4-Difluorobenzyl)-3-[3-fluoro-2-(trifluorométhyl)phényl]-8-méthoxycinnolin-4(1*H*)-one ;
- 1-(2,4-Difluorobenzyl)-8-méthoxy-3-[3-(trifluorométhoxy)phényl]cinnolin-4(*H*)-one ;
- 4-{[3-(2,3-Dichlorophényl)-4-oxocinnolin-1(4*H*)-yl]méthyl}-3-fluorobenzonitrile ;
- 3-Fluoro-4-({3-[2-fluoro-3-(trifluorométhyl)phényl]-4-oxocinnolin-1(4*H*)-yl}méthyl)benzonitrile ;
- 1-(2,4-Difluorobenzyl)-3-(4-fluoro-2-méthoxyphényl)cinnolin-4(*H*)-one ;
- 3-(2,3-Dichlorophényl)-1-(2,4-difluorobenzyl)cinnolin-4(1*H*)-one ;
- 1-(2,4-Difluorobenzyl)-3-(5-fluoro-2-méthoxyphényl)cinnolin-4(1*H*)-one ;
- 1-(2,4-Difluorobenzyl)-3-(2-méthoxyphényl)cinnolin-4(1*H*)-one ;
- 3-(3-bromo-2-fluorophényl)-1-(2,4-difluorobenzyl)cinnolin-4(1*H*)-one
- 1-(2,4-Difluorobenzyl)-3-(2-fluoro-3-méthylphényl)cinnolin-4(1*H*)-one ;
- 1-(2,4-Difluorobenzyl)-3-[2-(trifluorométhyl)phényl]cinnolin-4(1*H*)-one ;
- 1-(2,4-Difluorobenzyl)-3-[2-(trifluorométhoxy)phényl]cinnolin-4(1*H*)-one ;
- 1-(2,4-Difluorobenzyl)-3-[3-(trifluorométhoxy)phényl]cinnolin-4(1*H*)-one ;
- 1-(2-Chloro-4-fluorobenzyl)-3-[2-fluoro-3-(trifluorométhyl)phényl]cinnolin-4(1*H*)-one ;
- 1-(4-Chloro-2-fluorobenzyl)-3-(5-fluoro-2-méthoxyphényl)cinnolin-4(1*H*)-one ;
- 3-(3-Chloro-2-fluorophényl)-1-(2,4-difluorobenzyl)cinnolin-4(1*H*)-one ;
- 3-Fluoro-4-({4-oxo-3-[2-(trifluorométhyl)phényl]cinnolin-1(4*H*)-yl}méthyl)benzonitrile ;
- 1-(2,4-Difluorobenzyl)-3-(4-méthylphényl)cinnolin-4(1*H*)-one ;
- 1-(2,4-Difluorobenzyl)-3-[3-(trifluorométhyl)phényl]cinnolin-4(1*H*)-one ;
- 1-[(5-Chloro-2-thiényl)méthyl]-3-[2-fluoro-3-(trifluorométhyl)phényl]cinnolin-4(1*H*)-one ;
- 1-(2,4-Difluorobenzyl)-3-[2-(trifluorométhyl)pyridin-3-yl]cinnolin-4(1*H*)-one ;
- 3-(1-Benzothièn-7-yl)-1-(2,4-difluorobenzyl)cinnolin-4(1*H*)-one ;
- 1-Benzyl-3-(3-isopropylphényl)cinnolin-4(1*H*)-one ;
- 1-(2,4-Difluorobenzyl)-3-(5-isopropyl-2-méthoxyphényl)cinnolin-4(1*H*)-one ;
- 1-(2,4-Difluorobenzyl)-3-(1-naphthyl)cinnolin-4(1*H*)-one ;
- 1-(2,4-Difluorobenzyl)-3-[2-(trifluorométhyl)pyridin-3-yl]cinnolin-4(1*H*)-one ;
- 1-(2,4-Difluorobenzyl)-3-[2-fluoro-5-(trifluorométhoxy)phényl]cinnolin-4(1*H*)-one ;
- 1-(2,4-Difluorobenzyl)-3-(4-méthoxyphényl)cinnolin-4(1*H*)-one ;
- 3-(2,2-Difluoro-1,3-benzodioxol-4-yl)-1-(2,4-difluorobenzyl)cinnolin-4(1*H*)-one ;
- 3-(4-Chlorophényl)-1-(2,4-difluorobenzyl)cinnolin-4(1*H*)-one ;
- 1-[(5-Chloro-2-thiényl)méthyl]-3-(2,3-dichlorophényl)cinnolin-4(1*H*)-one ;
- 1-(4-Chloro-2-fluorobenzyl)-3-[3-(trifluorométhyl)phényl]cinnolin-4(1*H*)-one ;
- 1-(4-Fluorobenzyl)-3-(5-fluoro-2-méthoxyphényl)cinnolin-4(1*H*)-one ;
- 1-(2-Fluoro-4-méthylbenzyl)-3-[2-fluoro-3-(trifluorométhyl)phényl]cinnolin-4(1*H*)-one ;
- 1-(2-Fluorobenzyl)-3-(5-fluoro-2-méthoxyphényl)cinnolin-4(1*H*)-one ;
- 1-(2,4-Difluorobenzyl)-3-(2-fluoro-4-méthylphényl)cinnolin-4(1*H*)-one ;
- 1-(4-Fluorobenzyl)-3-[3-(trifluorométhyl)phényl]cinnolin-4(1*H*)-one ;
- 1-(2,4-Difluorobenzyl)-3-(3-fluorophényl)cinnolin-4(1*H*)-one ;
- 3-(5-Fluoro-2-méthoxyphényl)-1-[2-fluoro-4-(trifluorométhyl)benzyl]cinnolin-4(1*H*)-one ;
- 1-(4-Fluorobenzyl)-3-[2-fluoro-3-(trifluorométhyl)phényl]cinnolin-4(1*H*)-one ;
- 3-(5-Fluoro-2-méthoxyphényl)-1-(2-fluoro-4-méthylbenzyl)cinnolin-4(1*H*)-one ;
- 1-(2-Chloro-4-fluorobenzyl)-3-(5-fluoro-2-méthoxyphényl)cinnolin-4(1*H*)-one ;
- 1-(2,4-Difluorobenzyl)-3-(2,5-difluorophényl)cinnolin-4(1*H*)-one ;
- 1-(2,4-Difluorobenzyl)-3-[3-fluoro-5-(trifluorométhyl)phényl]cinnolin-4(1*H*)-one ;
- 1-(2,4-Difluorobenzyl)-3-(2-méthylphényl)cinnolin-4(1*H*)-one ;
- 1-(2,4-Difluorobenzyl)-3-(2-naphthyl)cinnolin-4(1*H*)-one ;
- 1-(2,4-Difluorobenzyl)-3-(2,3-difluorophényl)cinnolin-4(1*H*)-one ;
- 1-[2-Fluoro-4-(trifluorométhyl)benzyl]-3-[2-fluoro-3-(trifluorométhyl)phényl]cinnolin-4(1*H*)-one ;
- 3-[3-Chloro-5-(trifluorométhyl)phényl]-1-(2,4-difluorobenzyl)cinnolin-4(1*H*)-one ;
- 3-(3,5-Dichlorophenyl)-1-(2,4-difluorobenzyl)cinnolin-4(1*H*)-one ;
- 1-(2,4-Difluorobenzyl)-3-(2-fluorophényl)cinnolin-4(1*H*)-one ;
- 1-(2,4-Dichlorobenzyl)-3-(5-fluoro-2-méthoxyphényl)cinnolin-4(1*H*)-one ;
- 3-[2-Fluoro-3-(trifluorométhyl)phényl]-1-{[5-(trifluorométhyl)pyridin-2-yl]méthyl}cinnolin-4(1*H*)-one ;
- 3-(5-*tert*-butyl-2-méthoxyphenyl)-1-(2,4-difluorobenzyl)cinnolin-4(1*H*)-one ;
- 1-[2-Fluoro-4-(trifluorométhyl)benzyl]-3-[3-(trifluorométhyl)phényl]cinnolin-4(1*H*)-one ;
- 3-[4-Chloro-2-(trifluorométhyl)phényl]-1-(2,4-difluorobenzyl)cinnolin-4(1*H*)-one ;
- 1-(2,4-Fifluorobenzyl)-3-(5-fluoro-2-hydroxyphényl)cinnolin-4(1*H*)-one ;
- 1-(2,4-Difluorobenzyl)-3-phénylcinnolin-4(1*H*)-one ;
- 1-(2,4-Dichlorobenzyl)-3-[3-(trifluorométhyl)phényl]cinnolin-4(1*H*)-one ;
- 1-(2-Chloro-4-fluorobenzyl)-3-[3-(trifluorométhyl)phényl]cinnolin-4(1*H*)-one ;
- 1-(2-Fluoro-4-methylbenzyl)-3-[3-(trifluorométhyl)phényl]cinnolin-4(1*H*)-one ;
- 3-(1-Benzothien-2-yl)-1-(4-chlorobenzyl)cinnolin-4(1*H*)-one ;
- 1-(2-Fluorobenzyl)-3-[2-fluoro-3-(trifluorométhyl)phényl]cinnolin-4(1*H*)-one ;
- 1-[(5-Chloro-2-thiényl)méthyl]-3-(5-fluoro-2-méthoxyphényl)cinnolin-4(1*H*)-one ;
- 1-(2,4-Difluorobenzyl)-3-[4-(trifluorométhoxy)phényl]cinnolin-4(1*H*)-one;
- 1-(2,4-Difluorobenzyl)-3-(6-méthoxypyridin-3-yl)cinnolin-4(1*H*)-one;
- 3-(2,3-Dichlorophényl)-1-{[5-(trifluorométhyl)pyridin-2-yl]méthyl}cinnolin-4(1*H*)-one ;
- 7-Chloro-1-(2,4-difluorobenzyl)-3-[2-fluoro-3-(trifluorométhyl)phényl]cinnolin-4(1*H*)-one ;
- 1-(2,4-Difluorobenzyl)-3-[3-fluoro-2-(trifluorométhyl)phényl]-7-méthoxycinnolin-4(1*H*)-one ;
- 7-Chloro-3-[2-chloro-3-(trifluorométhyl)phényl]-1-(2,4-difluorobenzyl)cinnolin-4(1*H*)-one ;
- 1-(2,4-Difluorobenzyl)-7-méthoxy-3-[3-(trifluorométhoxy)phényl]cinnolin-4(1*H*)-one ;
- 4-({3-[2-Chloro-3-(trifluorométhyl)phényl]-4-oxocinnolin-1(4*H*)-yl}méthyl)-3-fluorobenzonitrile ;
- 7-Chloro-1-(2,4-difluorobenzyl)-3-[3-fluoro-2-(trifluorométhyl)phényl]cinnolin-4(1*H*)-one ;
- 7-Chloro-3-(2,3-dichlorophényl)-1-(2,4-difluorobenzyl)cinnolin-4(1*H*)-one ;
- 7-Chloro-1-(2,4-difluorobenzyl)-3-[3-(trifluorométhoxy)phényl]cinnolin-4(1*H*)-one ;
- 7-Chloro-1-(2,4-difluorobenzyl)-3-[2-(méthylsulfonyl)phényl]cinnolin-4(1*H*)-one ;
- 1-[(5-Chloropyridin-2-yl)méthyl]-3-[2-fluoro-3-(trifluorométhyl)phényl]cinnolin-4(1*H*)-one ;
- 6-Chloro-3-[2-chloro-3-(trifluorométhyl)phényl]-1-(2,4-difluorobenzyl)cinnolin-4(1*H*)-one ;
- 6-Chloro-1-(2,4-difluorobenzyl)-3-[2-fluoro-3-(trifluorométhyl)phényl]cinnolin-4(1*H*)-one ;
- 1-(2,4-Difluorobenzyl)-3-[2-fluoro-3-(trifluorométhyl)phényl]-6-méthoxycinnolin-4(1*H*)-one ;
- 6-Chloro-1-[(3,5-difluoropyridin-2-yl)méthyl]-3-[2-fluoro-3-(trifluorométhyl)phényl]cinnolin-4(1*H*)-one ;
   à l'état de base ou de sel d'addition à un acide.

Dans ce qui suit, on entend par groupe protecteur GP un groupe qui permet, d'une part, de protéger une fonction réactive telle qu'un hydroxy ou une amine pendant une synthèse et, d'autre part, de régénérer la fonction réactive intacte en fin de synthèse. Des exemples de groupes protecteurs ainsi que des méthodes de protection et de déprotection sont données dans "Protective Group in Organic Synthesis", Green et al., 4th Edition (John Wiley & Sons, Inc., New York), 2007.

On entend par groupe partant, dans ce qui suit, un groupe pouvant être facilement clivé d'une molécule par rupture d'une liaison hétérolytique, avec départ d'une paire électronique. Ce groupe peut ainsi être remplacé facilement par un autre groupe lors d'une réaction de substitution, par exemple. De tels groupes partants sont, par exemple, les halogènes ou un groupe hydroxy activé tel qu'un méthanesulfonate, benzènesulfonate, p-toluènesulfonate, triflate, acétate, etc. Des exemples de groupes partants ainsi que des références pour leur préparation sont donnés dans "Advanced Organic Chemistry", M.B. Smith and J. March, 6th Edition, Wiley Interscience, 2007, p. 496-501.

Conformément à l'invention, on peut préparer les composés de formule (I) selon un procédé qui est caractérisé en ce que :
on fait réagir en présence d'une base, un composé de formule : 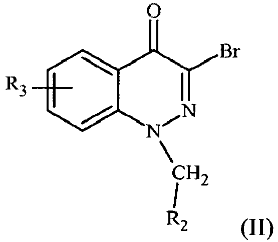 dans laquelle R₂ et R₃ sont tels que définis pour un composé de formule (I), avec un composé de formule :

   R₁-B(OH)₂ (III)
dans laquelle R₁ est tel que défini pour un composé de formule (I).

La réaction s'effectue en présence d'un catalyseur au palladium tel que par exemple, le tetrakis(triphénylphosphine)palladium, en présence d'une base telle que par exemple, le carbonate de sodium, dans un solvant tel que par exemple, le toluène, le méthanol ou un mélange de ces deux solvants, à une température comprise entre la température ambiante et 100°C.

Eventuellement, on transforme le composé de formule (I) en l'un de ses sels d'addition avec des acides minéraux ou organiques.

Les composés de formule (I) ainsi obtenus peuvent être ultérieurement séparés du milieu réactionnel et purifiés selon les méthodes classiques, par exemple par cristallisation ou chromatographie.

Les composés de formule (I) ainsi obtenus sont isolés sous forme de base libre ou de sel, selon les techniques classiques.

Les composés de formule (II) se préparent par réaction d'un composé de formule : 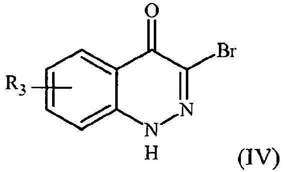 dans laquelle R₃ est tel que défini pour un composé de formule (I) avec un composé de formule:

Hal-CH₂-R₂ (V)

dans laquelle R₂ est tel que défini pour un composé de formule (I) et Hal représente un atome d'halogène.

La réaction s'effectue en présence d'une base forte telle que par exemple, l'hydrure de sodium, dans un solvant tel que par exemple, le N,N-diméthylformamide à une température comprise entre 0° et la température de reflux du solvant.

Les composés de formule (III) sont commerciaux, connus ou préparés selon des méthodes connues de l'homme de l'art.

Les composés de formule (IV) se préparent à partir d'un composé de formule : 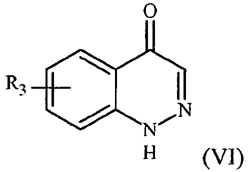 dans laquelle R₃ est tel que défini pour un composé de formule (I), par réaction avec du brome, en présence d'une base telle que par exemple, l'éthylate de potassium, dans un solvant tel que par exemple, l'acide acétique et à une température comprise entre la température ambiante et la température de reflux du solvant.

Les composés de formule (V) sont commerciaux, connus ou préparés selon des méthodes connues de l'homme de l'art.

Les composés de formule (VI) se préparent par cyclisation d'un composé de formule : 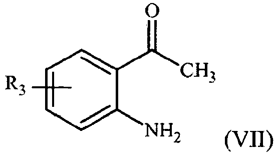 dans laquelle R₃ est tel que défini pour un composé de formule (I), en présence de nitrite de sodium, dans un solvant tel que par exemple, l'acide chlorhydrique et à une température comprise entre 0°C et la température ambiante.

Les composés de formule (VII) dans laquelle R₃ est tel que défini pour un composé de formule (I) se préparent selon le SCHEMA I ci-après.

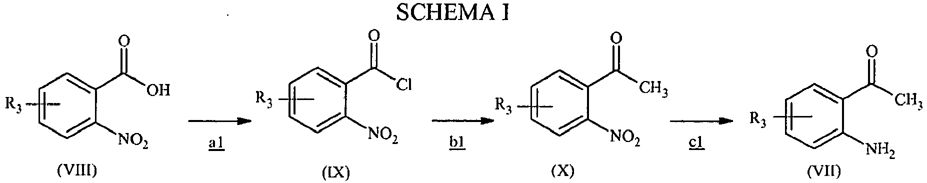

A l'étape a1 du SCHEMA I, la réaction d'un composé de formule (VIII) avec du chlorure de sulfonyle s'effectue à une température comprise entre la température ambiante et 100°C.

A l'étape b1, le composé de formule (IX) ainsi obtenu est mis en réaction avec du malonate de diéthyle, en présence de magnésium, dans un solvant tel que par exemple, un éther (éther diéthylique par exemple) à une température comprise entre la température ambiante et la température de reflux du solvant.

Le composé (X) ainsi obtenu est réduit à l'étape c1 en présence de zinc, d'acide acétique dans un solvant tel que par exemple, le tétrahydrofurane et à une température comprise entre 0°C et la température ambiante.

Le composé de formule (VIII) est commercial, connu ou préparé selon des méthodes connues de l'homme de l'art.

Selon une variante de ce procédé, on peut également préparer un composé de formule (VII) dans laquelle R₃ est tel que défini pour un composé de formule (I) selon le SCHEMA II.

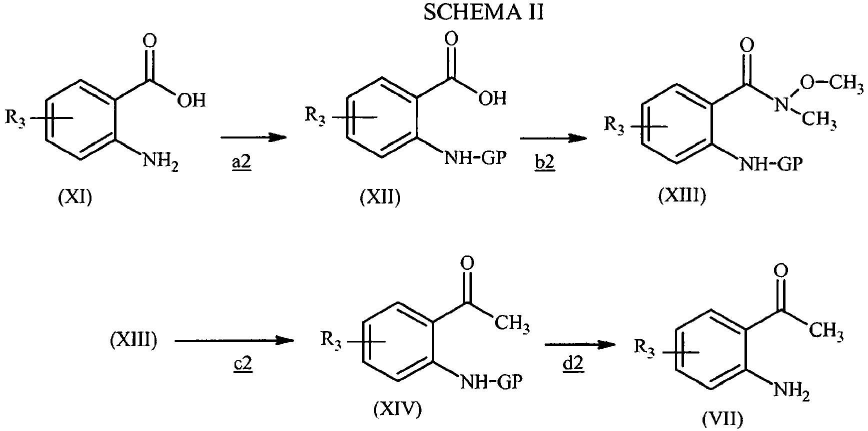

A l'étape a2 du SCHEMA II, on protège l'amine du composé formule (XI) selon les méthodes connues de l'homme du métier.

A l'étape b2, le composé de formule (XII) ainsi obtenu est mis en réaction avec la N-méthoxyméthanamine, en présence d'un agent de couplage utilisé en chimie peptidique tel que par exemple, le 1,3-dicyclohexylcarbodiimide (DCC) ou l'hexafluorophosphate de benzotriazol-1-yloxytris(diméthylamino) phosphonium (BOP) ou l'hexafluorophosphate de benzotriazol-1-yloxytris(pyrrolidino) phosphonium (PyBOP) ou le tétrafluoroborate de 2-(1*H*-benzotriazol-1-yl)-1,1,3,3-tétraméthyl uronium, en présence d'une base telle que par exemple, la triéthylamine, la N,N-diisopropyléthyl amine ou la 4-diméthylaminopyridine, dans un solvant tel que par exemple, le dichlorométhane, le dichloroéthane, le N-N-diméthylformamide ou le tétrahydrofurane à une température comprise entre -10°C et la température de reflux du solvant.

Le composé (XIII) ainsi obtenu est mis à réagir à l'étape c2 avec un composé organométallique tel que par exemple, le bromure de méthylmagnésium dans un solvant tel que par exemple, un éther (tétrahydrofurane, dioxane par exemple) à une température comprise entre -100°C et la température ambiante.

Le composé (XIV) ainsi obtenu est déprotégé à l'étape d2.

Les EXEMPLES suivants décrivent la préparation de certains composés conformes à l'invention. Ces exemples ne sont pas limitatifs et ne font qu'illustrer la présente invention. Les numéros des composés exemplifiés renvoient à ceux donnés dans le TABLEAU I ci-après, qui illustre les structures chimiques et les propriétés physiques de quelques composés selon l'invention.

Dans les Préparations et dans les Exemples on utilise les abréviations suivantes :
éther : éther diéthylique
éther iso : éther diisopropylique
DMSO : diméthylsulfoxyde
DMF : N,N-diméthylformamide
THF : tétrahydrofurane
MeOH : méthanol
DCM : dichlorométhane
AcOEt : acétate d'éthyle
HBr : acide bromhydrique
AcONa : acétate de sodium
DIPEA : diisopropyléthylamine
TFA : acide trifluoroacétique
F : point de fusion
TA : température ambiante
CLHP : chromatographie liquide haute performance

Les spectres de résonance magnétique nucléaire du proton (RMN ¹H) sont enregistrés à 200 MHz dans le DMSO-d₆. Les déplacements chimiques δ sont exprimés en parties par million (ppm). Pour l'interprétation des spectres, on utilise les abréviations suivantes : s : singulet, d : doublet, t : triplet, qd : quadruplet, m : massif, mt : multiplet, se : singulet élargi, dd : doublet dédoublé.

Les mélanges de solvants sont quantifiés en rapport volumétriques.

Les composés selon l'invention sont analysés par couplage LC/UV/MS (Chromatographie Liquide/ détection UV/Spectrométrie de masse). On mesure le pic moléculaire (MH⁺) et le temps de rétention (tr) en minutes.

Les conditions utilisées sont les suivantes :

### Conditions A

Colonne : Symmetry C₁₈ (2,1 x 50 mm) 3,5 µm ;
Eluant : A : H₂O + 0,005 % TFA pH ≈ 3 ;
B : acétonitrile / 0,005 % TFA ;

### Gradient :

| Temps (min.) | % A | % B |
|---|---|---|
| 0 | 100 | 0 |
| 10 | 10 | 90 |
| 15 | 10 | 90 |
| 16 | 0 | 100 |
| 20 | 0 | 100 |

Débit : 0,4 ml/minute ;
Détection UV : λ = 210-220 nm ;

### Conditions B

On utilise une colonne XTerra MS C₁₈ (2,1 x 50 mm) 3,5 µm ;
Eluant : A : 10mM AcONH₄ pH ≈ 7 ;
B : acétonitrile ;

### Gradient :

| Temps (mn) | % A | % B |
|---|---|---|
| 0 | 100 | 0 |
| 10 | 10 | 90 |
| 15 | 10 | 90 |
| 16 | 100 | 0 |
| 20 | 100 | 0 |

Débit : 0,4 ml/minute ;
Détection UV : λ = 220 nm ;

### Conditions C

On utilise une colonne Acquity BEH C₁₈ (2,1 x 50 mm) 1,7 µm ;
Eluant : A : H₂O + 0,05 % TFA pH ≈ 3 ; acétonitrile (97/3)
B : acétonitrile / 0,035 % TFA ;

### Gradient :

| Temps (mn) | % A | % B |
|---|---|---|
| 0 | 100 | 0 |
| 2,3 | 5 | 95 |
| 2,9 | 5 | 95 |
| 3 | 100 | 0 |
| 3,5 | 100 | 0 |

Débit : 1 ml/minute ;
Détection UV : λ = 220 nm ;

### Conditions D

On utilise une colonne Kromasil C₁₈ (2 x 50 mm) 3,5 µm ;
Eluant : A : H₂O + 0,05 % TFA ;
B : acétonitrile / 0,035 % TFA ;

### Gradient :

| Temps (mn) | % A | % B |
|---|---|---|
| 0 | 95 | 5 |
| 6 | 0 | 100 |
| 8 | 0 | 100 |
| 8,1 | 95 | 0 |

Débit : 0,7 ml/minute ;
Détection UV : λ = 220 nm ;

L'enregistrement des spectres de masse est effectué en mode electrospray (ESI) positif, afin d'observer les ions issus de la protonation des composés analysés (MH⁺) ou de la formation d'adduits avec d'autres cations tels que N⁺, K⁺, etc....

### PREPARATIONS

### 1. Préparations des composés de formule (VII).

### Préparation 1.1

1-(2-Amino-3-méthoxyphényl)éthanone.

(VII) : R₃ = 3-OMe.

### A- Chlorure de 3-méthoxy-2-nitrobenzoyle.

On chauffe à 75°C pendant 3 heures un mélange de 10 g d'acide 3-méthoxy-2-nitrobenzoïque dans 60 ml de chlorure de thionyle. On concentre le mélange réactionnel sous vide et obtient 10,9 g du composé attendu que l'on utilise tel quel à l'étape suivante.

### B- 1-(3-méthoxy-2-nitrophényl)éthanone.

A un mélange de 3,2 g de magnésium dans 10 ml d'éther on ajoute, goutte à goutte et à TA, une solution de 2 ml de malonate de diéthyle dans 1 ml d'EtOH puis chauffe à reflux. On ajoute ensuite, goutte à goutte, une solution de 18 ml de malonate de diéthyle dans 10 ml d'EtOH puis, goutte à goutte, une solution de 10,9 g du composé de l'étape précédente dans 40 ml d'éther et chauffe à reflux pendant 18 heures. On verse le mélange réactionnel dans 100 ml d'éther et filtre l'insoluble. On reprend l'insoluble par une solution saturée de NH₄Cl, extrait avec 100 ml de chloroforme, acidifie la phase aqueuse par ajout de 20 ml d'une solution d'HCl à 10 %, reextrait la phase aqueuse avec 100 ml de chloroforme, séche les phases organiques jointes sur MgSO₄ et évapore les solvants sous vide. On reprend le résidu dans un mélange de 10 ml d'acide acétique, 1,5 ml d'H₂SO₄ et 7 ml d'eau puis chauffe à reflux pendant 5 heures. On concentre l'acide acétique sous vide, reprend le mélange réactionnel par 100 ml d'eau, alcalinise la phase aqueuse par ajout de NH₄OH, extrait avec 100 ml de chloroforme, lave la phase organique par une solution saturée de NaCl, séche sur MgSO₄ et évapore le solvant sous vide. On chromatographie le résidu sur gel de silice en éluant par le gradient du mélange cyclohexane/AcOEt de (80/20; v/v) à (20/80; v/v). On obtient 3,3 g du composé attendu.

### C- 1-(2-Amino-3-méthoxyphényl)éthanone.

On refroidit à 0°C une solution de 3,3 g du composé de l'étape précédente dans 100 ml de THF, ajoute 13,27 g de zinc et 15 ml d'acide acétique puis laisse sous agitation en laissant remonter la température à TA et laisse 4 heures sous agitation à TA. On filtre le mélange réactionnel sur Célite® et concentre le filtrat sous vide. On extrait le résidu au THF, lave la phase organique par 100 ml d'une solution de NaOH à 10%, par une solution saturée de NaCl, séche sur MgSO₄ et évapore le solvant sous vide. On chromatographie le résidu sur gel de silice en éluant par le gradient du mélange cyclohexane/AcOEt de (80/20;v/v) à (60/40;v/v). On obtient 2 g du composé attendu.

### Préparation 1.2

1-(2-Amino-4-chlorophényl)éthanone.

(VII) : R₃ = 4-Cl.

### A- Acide 2-[(tert-butoxycarbonyl)amino]-4-chlorobenzoïque.

A un mélange de 10 g d'acide 2-amino-4-chlorobenzoïque dans 45 ml de dioxane et 15 ml d'eau on ajoute 19,5 ml de triéthylamine puis, goutte à goutte, une solution de 14 g de di-*tert*-butyl dicarbonate dans 30 ml de dioxane et laisse 48 heures sous agitation à TA. On concentre le mélange réactionnel sous vide, reprend le résidu dans un mélange eau/AcOEt, décante, lave la phase organique par une solution saturée de NaCl, séche sur MgSO₄ et évapore le solvant sous vide. On chromatographie le résidu sur gel de silice en éluant par le gradient du mélange AcOEt / cyclohexane On obtient 5,35 g du composé attendu.

### B- [5-chloro-2-[méthoxy(méthyl)carbamoyl]phényl]carbamate de tert-butyle.

A une solution de 5,35 g du composé de l'étape précédente dans 200 ml de DCM on ajoute 8,78 ml de triéthylamine puis 2,11 g de N-méthoxyméthanamine et 11,27 g de PyBOP et laisse une nuit sous agitation à TA. On lave le mélange réactionnel à l'eau, par une solution de NaOH à 10%, par une solution saturée de NaCl, séche sur MgSO₄ et évapore le solvant sous vide. On chromatographie le résidu sur gel de silice en éluant par le gradient du mélange cyclohexane/AcOEt de (80/20;v/v) à (60/40;v/v). On obtient 5,7 g du composé attendu.

### C- (2-Acétyl-5-chlorophényl)carbamate de tert butyle.

On refroidit à - 40°C une solution de 5,7 g du composé de l'étape précédente dans 445 ml de THF, ajoute, goutte à goutte, 38,8 ml de bromure de méthylmagnésium et laisse une nuit sous agitation à TA. On verse le mélange réactionnel sur une solution d'HCl à 10%, extrait à l'AcOEt, lave la phase organique par une solution saturée de NaCl, séche sur MgSO₄ et évapore sous vide le solvant. On chromatographie le résidu sur gel de silice en éluant par le mélange cyclohexane/AcOEt (80/20;v/v) puis à l'AcOEt. On obtient 2,05 g du composé attendu.

### D- 1-(2-Amino-4-chlorophényl)éthanone.

A une solution de 0,9 g du composé de l'étape précédente dans 17 ml de DCM on ajoute, goutte à goutte, 2,55 ml d'acide trifluoroacétique et laisse une nuit sous agitation à TA. On lave le mélange réactionnel par une solution de NaOH à 10%, par une solution saturée de NaCl, séche sur MgSO₄ et évapore le solvant. On obtient 0,5 g du composé attendu.

### 2. Préparations des composés de formule (VI).

### Préparation 2.1

8-Méthoxycinnolin-4(1*H*)-one.

(VI): R₃ = 8-OMe.

On refroidit à 0°C un mélange de 2,0 g du composé de la Préparation 1.1 dans 8 ml d'HCl concentré, ajoute, goutte à goutte et en maintenant la température du mélange réactionnel en dessous de 10°C, une solution de 1,25 g de NaNO₂ dans 2,7 ml d'eau, laisse 2 heures sous agitation à 0°C et une nuit à TA. On concentre le mélange réactionnel sous vide, ajoute 50 ml d'une solution d'acétate de sodium, essore le précipité formé et le lave avec 20 ml d'eau. On obtient 1,95 g du composé attendu.

### Préparation 2.2

7-chlorocinnolin-4(1*H*)-one

(VI): R₃ = 7-Cl.

On refroidit à 0°C un mélange de 2,2 g du composé de la Préparation 1.2 dans 10 ml d'HCl concentré, ajoute goutte à goutte et en maintenant la température du mélange réactionnel en dessous de 10°C, une solution de 1,34 g de NaNO₂ dans 3 ml d'eau, laisse 2 heures sous agitation à 0°C et une nuit à TA. On concentre le mélange réactionnel sous vide, ajoute une solution d'acétate de sodium, essore le précipité formé et le lave. On obtient 1,1 g du composé attendu.

### 3. Préparations des composés de formule (IV).

### Préparation 3.1

3-Bromo-8-méthoxycinnolin-4(1H)-one.

(IV) : R₃ = 8-OMe; Hal = Br.

A un mélange de 1,95 g du composé de la Préparation 2.1 dans 20 ml d'acide acétique on ajoute 1,4 g d'éthylate de potassium et chauffe à reflux. On ajoute ensuite , goutte à goutte, une solution de 0,68 ml de brome dans 2 ml d'acide acétique et chauffe à reflux pendant 1 heures 30 minutes. On concentre l'acide acétique sous vide, reprend le résidu à l'eau, essore le précipité formé, le lave à l'eau et le séche. On obtient 2,8 g du composé attendu.

### Préparation 3.2

3-Bromo-7-chlorocinnolin-4(1*H*)-one.

(IV) : R₃ = 7-Cl ; Hal = Br.

A un mélange de 0,9 g du composé de la Préparation 2.2 dans 8 ml d'acide acétique on ajoute 0,63 g d'éthylate de potassium et chauffe à reflux. On ajoute ensuite, goutte à goutte une solution de 0,38 ml de brome dans 2 ml d'acide acétique et chauffe à reflux pendant 3 heures. On concentre l'acide acétique sous vide, reprend le résidu à l'eau, essore le précipité formé et le sèche. On obtient 1,17 g du composé attendu.

### 4. Préparations des composés de formule (II).

### Préparation 4.1

3-Bromo-1-(2,4-difluorobenzyl)-8-méthoxycinnolin-4(1*H*)-one.

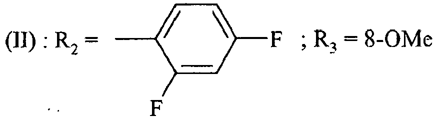

On refroidit à 0°C une suspension de 0,35 g de NaH à 60% dans l'huile dans 15 ml de DMF, ajoute, goutte à goutte, une solution de 1,5 g du composé de la Préparation 3.1 dans 15 ml de DMF puis une solution de 1,13 ml de 1-(bromométhyl)-2,4-difluorobenzène dans 10 ml de DMF et chauffe à 75°C pendant 3 heures. Après refroidissement à TA, on verse le mélange réactionnel sur 100 ml d'eau, extrait deux fois par 80 ml de DCM, lave la phase organique par une solution saturée de NaCl, sèche sur MgSO₄et évapore le solvant sous vide. On chromatographie le résidu sur gel de silice en éluant par le gradient du mélange cyclohexane/AcOEt de (80/20;v/v) à (50/50;v/v). On obtient 1,0 g du composé attendu.

### Préparation 4.2

### 3-Bromo-7-chloro-1-(2,4-difluorobenzyl)cinnolin-4(1H)-one.

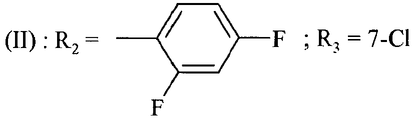

On refroidit à 0°C une suspension de 0,25 g de NaH à 60% dans l'huile dans 10 ml de DMF, ajoute, goutte à goutte, une solution de 1,1 g du composé de la Préparation 3.2 dans 15 ml de DMF puis une solution de 0,82 ml de 1-(bromométhyl)-2,4-difluorobenzène dans 5 ml de DMF et chauffe à 75°C pendant 3 heures. Après refroidissement à TA, on verse le mélange réactionnel dans l'eau, extrait au DCM, lave la phase organique par une solution saturée de NaCl, sèche sur MgSO₄ et évapore le solvant sous vide. On chromatographie le résidu sur gel de silice en éluant par le gradient du mélange cyclohexane/AcOEt de (80/20;v/v) à (50/50;v/v). On obtient 1,2 g du composé attendu.

### EXEMPLE 1 : Composé N° 2

### 3-[2-Chloro-3-(trifluorométhyl)phényl]-1-(2,4-difluorobenzyl)-8-méthoxycinnolin-4(1H)-one.

A une solution de 0,2 g du composé de la Préparation 4.1 dans un mélange de 8 ml de toluène et de 2 ml de MeOH on ajoute 0,14 g d'acide [2-chloro-3-(trifluorométhyl)phényl]boronique puis 0,87 ml d'une solution 2M de Na₂CO₃. On fait barboter de l'azote dans le mélange réactionnel pendant 20 minutes, ajoute 0,12 g de tétrakis (triphénylphosphine)palladium et chauffe à 80°C pendant 18 heures. On filtre le mélange réactionnel sur Célite® et lave à l'AcOEt. On lave le filtrat par 50 ml de NaOH à 50%, par une solution saturée de NaCl, sèche sur MgSO₄ et évapore les solvants sous vide. On chromatographie le résidu sur gel de silice en éluant par le gradient du mélange cyclohexane/AcOEt de (90/10;v/v) à (70/30;v/v). On obtient 0,2 g du composé attendu, F = 141-142 °C.
RMN ¹H : DMSO-d₆ (250 MHz) : δ (ppm) : 3.84 : s : 3 H ; 5.99 : s : 2 H ; 6.96-7.22 : m : 2 H ; 7.23 - 7.37 : m : 1 H ; 7.41 - 7.57 : m : 2 H ; 7.63 - 7.75 : m : 1 H ; 7.78 - 7.90 : m : 2 H ; 7.95 - 8.04 : m : 1 H.

### EXEMPLE 2 : Composé N° 76

### 7-Chloro-1-(2,4-difluorobenzyl)-3-[2-fluoro-3-(trifluorométhyl)phényl]cinnolin-4(1H)-one.

A une solution de 0,2 g du composé de la Préparation 4.2 dans un mélange de 8 ml de toluène et de 2 ml de MeOH on ajoute 0,13 g d'acide [2-fluoro-3-(trifluorométhyl)phényl]boronique puis 0,86 ml d'une solution 2M de Na₂CO₃. On fait barboter de l'azote dans le mélange réactionnel pendant 20 minutes, ajoute 0,12 g de tétrakis (triphénylphosphine)palladium et chauffe à 80°C pendant une nuit. On filtre le mélange réactionnel sur Célite® et concentre sous vide le filtrat. On extrait le résidu à l'AcOEt, lave la phase organique par une solution de NaOH à 10%, par une solution saturée de NaCl, sèche sur MgSO₄ et évapore les solvants sous vide. On chromatographie le résidu sur gel de silice en éluant par le gradient du mélange cyclohexane/AcOEt de (80/20;v/v) à (60/40;v/v). On obtient 0,16 g du composé attendu, F = 118-120 °C.

RMN ¹H : DMSO-d₆ (250 MHz) : δ (ppm) : 5.83 : s : 2 H ; 7.01 - 7.16 : m : 1 H ; 7.27 - 7.65 : m : 4 H ; 7.82 - 7.96 : m : 2 H ; 8.08 : d : 1 H ; 8.24 : d : 1 H.

Le tableau qui suit illustre les structures chimiques et les propriétés physiques de quelques exemples de composés selon l'invention, obtenus en suivant les modes opératoires décrits dans les Exemples ci-dessus.

Dans ce tableau :
- Me représente un groupe méthyle.

**TABLEAU I**

| | | | | |
|---|---|---|---|---|
| 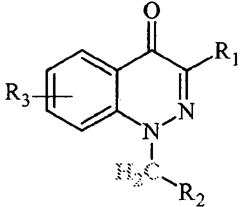 | | | | |

| **Composé No.** | **R₁** | **R₂** | **R₃** | **F°C ; MH⁺ ; tr (min.) conditions** |
|---|---|---|---|---|
| **1** | 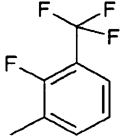 | 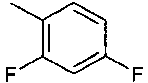 | 8-OMe | 116-118°C 465 ; 10,59 A |
| **2** | 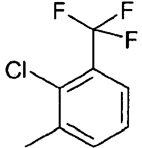 | 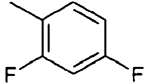 | 8-OMe | 141-142°C 481 ; 11,1 A |
| **3** | 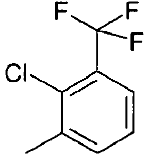 | 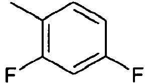 | H | 168-170°C 451 ; 10,62 A |
| **4** | 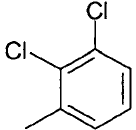 | 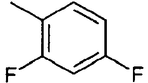 | 8-OMe | 103-105°C 447 ; 10,94 A RMN |
| **5** | 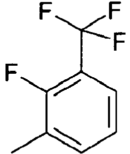 | 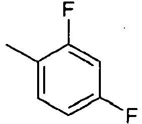 | H | 150-151°C 435 ; 10,60 A |
| **6** | 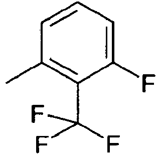 | 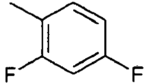 | H | 435 ; 10,21 A |
| **7** | 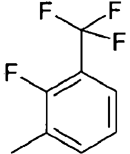 | 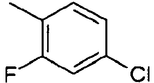 | H | 172-174°C 451 ; 11,07 A |
| **8** | 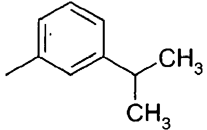 | 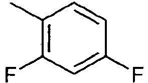 | H | 390 ; 5,4 D |
| **9** | 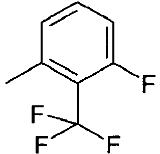 | 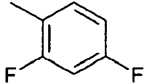 | 8-OMe | 135-136°C 465 ; 10,63 A |
| **10** | 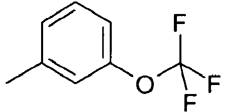 | 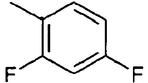 | 8-OMe | 153-155°C 463 ; 11,66 A |
| **11** | 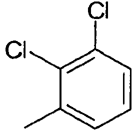 | 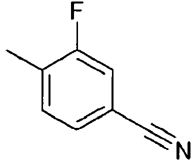 | H | 272°C 424 ; 9,90 A |
| **12** | 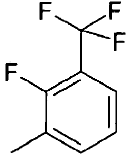 | 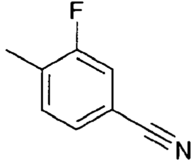 | H | 220°C 442 ; 10,05 A |
| **13** | 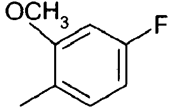 | 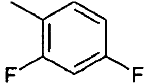 | H | 148-150°C 397 ; 9,66 A |
| **14** | 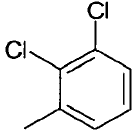 | 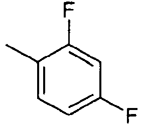 | H | 208-210°C 417 ; 10,47 A |
| **15** | 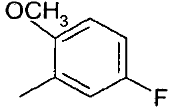 | 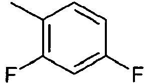 | H | 137-138°C 397 ; 9,66 A |
| **16** | 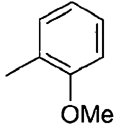 | 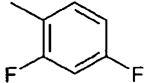 | H | 379 ; 434 D |
| **17** | 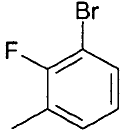 | 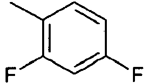 | H | 190-192°C 445 ; 37,1 A |
| **18** | 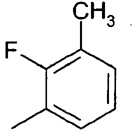 | 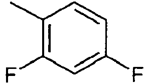 | H | 154-155°C 381 ; 10,09 A |
| **19** | 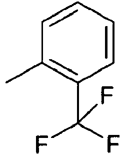 | 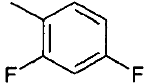 | H | 134-135°C 417 ; 10,07 A |
| **20** | 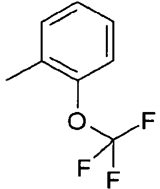 | 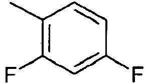 | H | 96-98°C 433 ; 10,35 A |
| **21** | 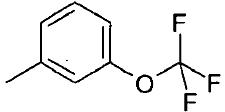 | 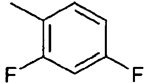 | H | 100-102°C 433 ; 11,20 A |
| **22** | 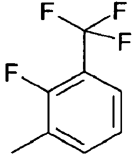 | 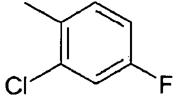 | H | 124-125°C 451 ; 11,1 A |
| **23** | 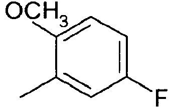 | 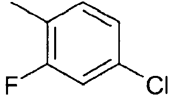 | H | 155-156°C 413 ; 10,13 A |
| **24** | 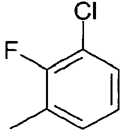 | 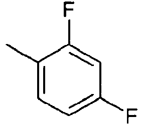 | H | 183-184°C 401 ; 9,92 B |
| **25** | 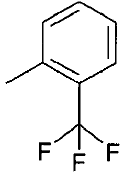 | 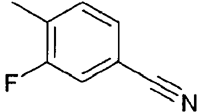 | H | 166-167°C 424 ; 9,5 A |
| **26** | 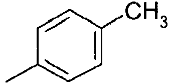 | 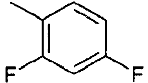 | H | 363 ; 4,95 D |
| **27** | 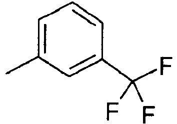 | 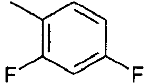 | H | 122-124°C 417 ; 11,05 A |
| **28** | 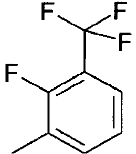 | 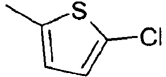 | H | 140°C 439 ; 10,92 A |
| **29** | 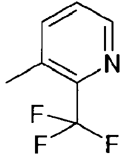 | 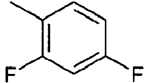 | H | 110-111°C 418 ; 1,69 C |
| **30** | 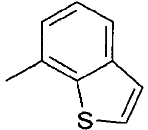 | 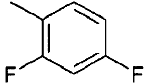 | H | 160-162°C 405 ; 10,86 A |
| **31** | 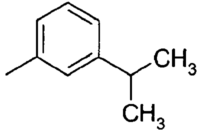 | 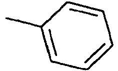 | H | 355 ; 4,94 D |
| **32** | 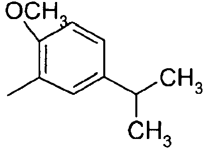 | 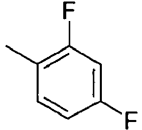 | H | 421 ; 4,77 D |
| **33** | 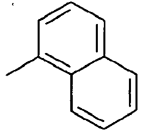 | 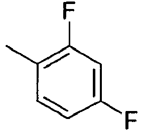 | H | 399 ; 4,83 D |
| **34** | 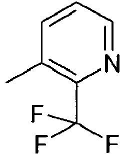 | 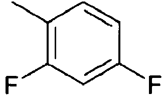 | H | 418 ; 1,69 C |
| **35** | 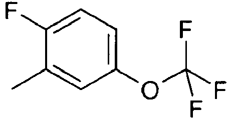 | 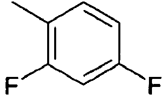 | H | 124-126°C 451 ; 10,7 A |
| **36** | 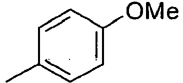 | 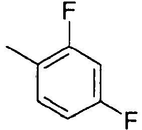 | H | 379 ; 4,61 D |
| **37** | 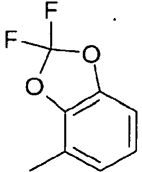 | 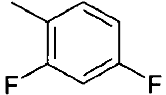 | H | 155-157°C 429 ; 10,69 A |
| **38** | 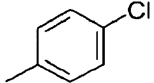 | 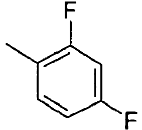 | H | 383 ; 5,15 D |
| **39** | 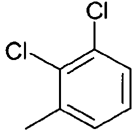 | 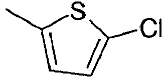 | H | 202-203°C 421 ; 10,78 A |
| **40** | 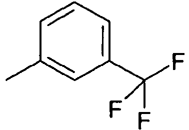 | 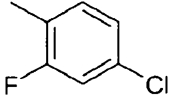 | H | 143-145°C 433 ; 11,5 A |
| **41** | 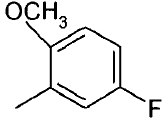 | 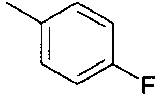 | H | 146-147°C 379 ; 9,43 A |
| **42** | 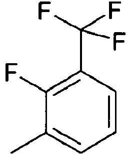 | 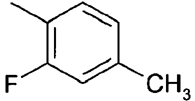 | H | 130-131°C 431 ; 10,87 A |
| **43** | 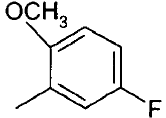 | 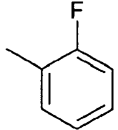 | H | 128-129°C 379 ; 9,51 A |
| **44** | 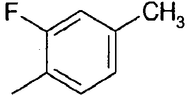 | 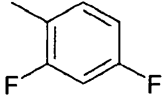 | H | 168-170°C 381 ; 10,16 A |
| **45** | 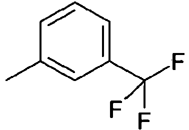 | 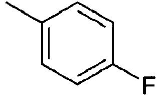 | H | 127-128°C 399 ; 10,9 A |
| **46** | 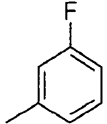 | 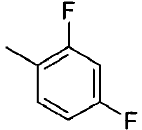 | H | 367 ; 4,9 D |
| **47** | 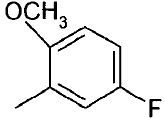 | 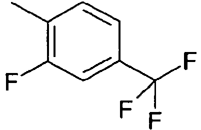 | H | 149-150°C 447 ; 10,29 A |
| **48** | 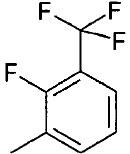 | 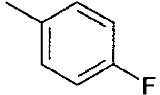 | H | 146-148°C 417; 10,4 A |
| **49** | 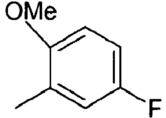 | 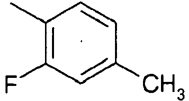 | H | 156-158°C 393; 10,0 A |
| **50** | 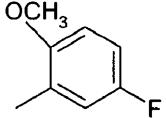 | 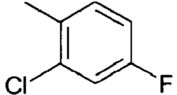 | H | 62-64°C 413 ; 10,18 A |
| **51** | 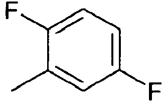 | 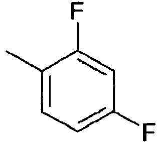 | H | 177-178°C 385 ; 9,93 A |
| **52** | 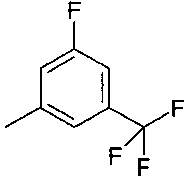 | 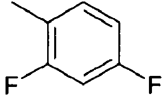 | H | 127-129°C 435 ; 11,41 A |
| **53** | 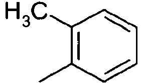 | 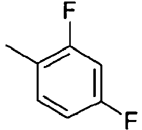 | H | 363 ; 4,51 D |
| **54** | 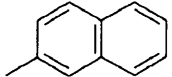 | 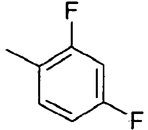 | H | 399 ; 5,24 D |
| **55** | 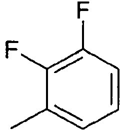 | 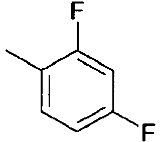 | H | 182-184°C 385 ; 9,96 A |
| **56** | 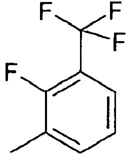 | 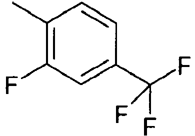 | H | 143-145°C 485 ; 11,09 A |
| **57** | 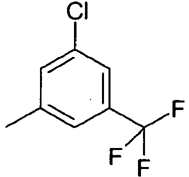 | 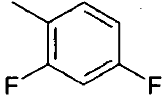 | H | 164-166°C 451 ; 19,17 A |
| **58** | 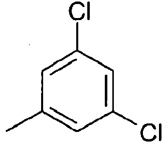 | 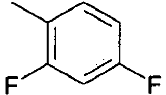 | H | 198-200°C 417; 11,37 B neutre |
| **59** | 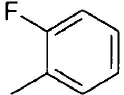 | 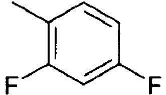 | H | 366 ; 33,5 A |
| **60** | 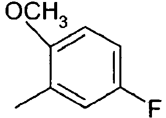 | 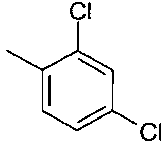 | H | 70-72°C 429-10,77 A |
| **61** | 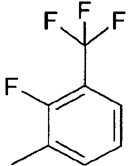 | 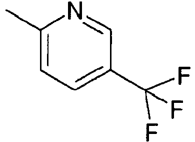 | H | 118°C 468 ; 10,43 A |
| **62** | 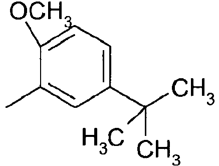 | 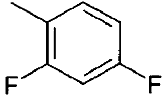 | H | 72-74°C 435 ; 10,92 A |
| **63** | 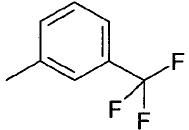 | 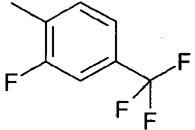 | H | 158-159°C 467 ; 11,55 A |
| **64** | 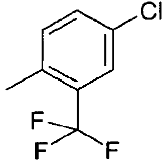 | 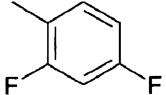 | H | 117-118°C 451 ; 10,78 A |
| **65** | 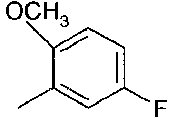 | 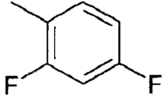 | H | 134-136°C 383 ; 10,09 A |
| **66** | 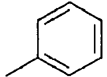 | 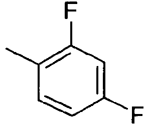 | H | 349 ; 4,78 D |
| **67** | 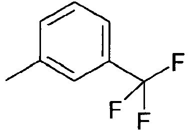 | 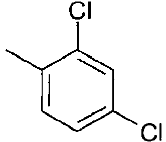 | H | 170-171°C 449 ; 12,06 A |
| **68** | 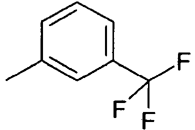 | 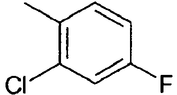 | H | 149-150°C 433 ; 11,53 A |
| **69** | 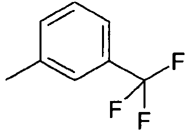 | 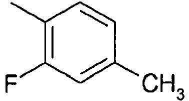 | H | 167-168°C 413 ; 11,46 A |
| **70** | 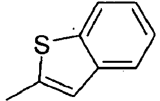 | 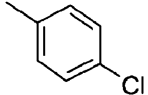 | H | 403 ; 5,75 D |
| **71** | 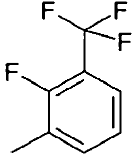 | 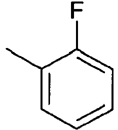 | H | 127-128°C 417 ; 10,47 A |
| **72** | 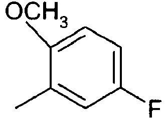 | 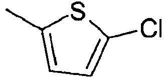 | H | 132-134°C 401 ; 9,9 A |
| **73** | 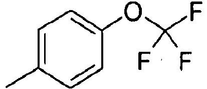 | 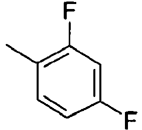 | H | 433 ; 5,37 D |
| **74** | 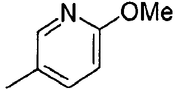 | 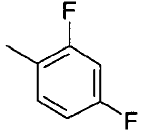 | H | 380 ; 4,24 D |
| **75** | 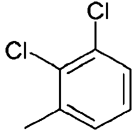 | 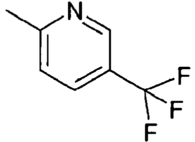 | H | 249°C 450 ; 10,22 A |
| **76** | 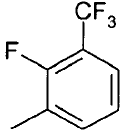 | 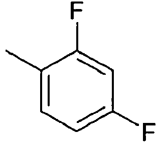 | 7-Cl | 118-120°C 469 ; 2 C |
| **77** | 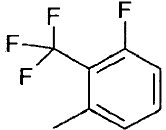 | 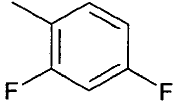 | 7-OMe | 177-178°C 465 ; 1,78 A |
| **78** | 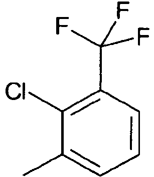 | 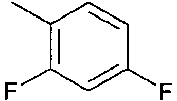 | 7-Cl | 196-198°C 485 ; 2,01 A |
| **79** | 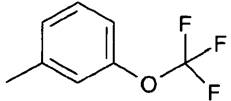 | 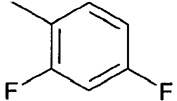 | 7-OMe | 160-162°C 463 ; 2,0 A |
| **80** | 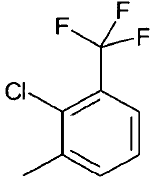 | 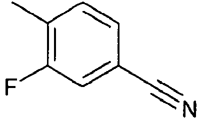 | H | 231°C 458 ; 9,59 A |
| **81** | 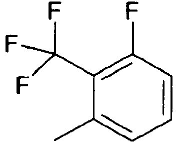 | 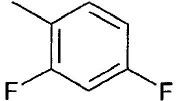 | 7-Cl | 160°C 469; 1,97 A |
| **82** | 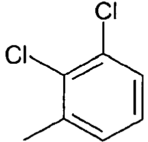 | 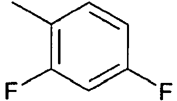 | 7-Cl | 212-214°C 451 ; 2,04 A |
| **83** | 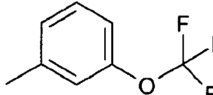 | 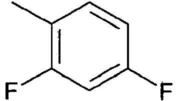 | 7-Cl | 137-138°C 467 ; 11,3 A |
| **84** | 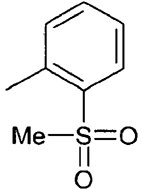 | 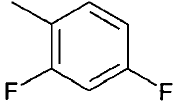 | 7-Cl | 196-197°C 461 ; 1,97 A |
| **85** | 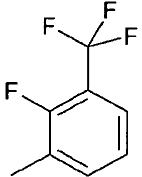 | 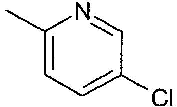 | H | 172-173°C 434 ; 1,76 A |
| **86** | 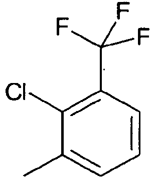 | 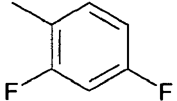 | 6-Cl | 166-168°C 485 ; 2,01 A |
| **87** | 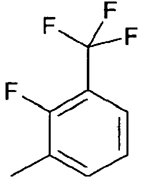 | 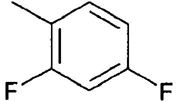 | 6-Cl | 118-120°C 469 ; 2,0 A |
| **88** | 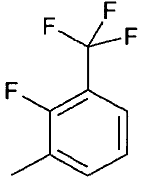 | 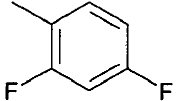 | 6-OMe | 178-180°C 465 ;. 1,88 A |
| **89** | 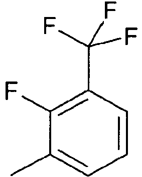 | 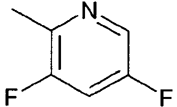 | 6-Cl | 136°C 470 ; 1,89 A |

Composé N° 4 : 1H NMR (250 MHz, DMSO-d6) d ppm 3.84 (s, 3 H), 5.98 (s, 2 H), 6.96 - 7.21 (m, 2 H), 7.23 - 7.36 (m, 1 H), 7.41 - 7.56 (m, 4 H), 7.73 - 7.85 (m, 2 H).

Les composés selon l'invention ont montré une très bonne affinité in vitro (C150 < 500 nM) pour les récepteurs CB₂ humain et de rongeur. Les essais de liaison par affinité (binding) ont été réalisés selon les conditions expérimentales décrites par M. Rinaldi-Carmona dans J. Pharmacol. Exp. Therap. 1998, 287, 644-650, avec des membranes issues soit de tissus de rongeur, soit de lignée cellulaires recombinantes dans lesquelles les récepteurs CB₂ humains ont été exprimés (Munro et al., Nature 1993, 365, 61-65). L'affinité des composés est exprimée sous forme de CI50 (concentration causant une inhibition de 50% de la liaison spécifique du ligand tritié utilisé in vitro).

Les composés selon l'invention ont montré un effet modulateur sur les récepteurs CB₂. Notamment, les composés selon l'invention présentent des propriétés de nature agoniste, agoniste inverse et/ou antagoniste.

La nature agoniste des composés selon l'invention a été démontrée dans les modèles d'inhibition de l'adénylate-cyclase (stimulée par de la forskoline) comme décrit dans M. Rinaldi-Carmona et al., J. Pharmacol. Exp. Ther. 1996, 278, 871-878 et 1998, 284, 644-650 et M Bouaboula et al., J. Biol Chem., 1997, 272, 22330-22339.

La nature antagoniste des composés selon l'invention a été démontrée dans les modèles de reversion de l'inhibition de l'adénylate-cyclase (stimulée par de la forskoline) induite par un agoniste des récepteurs CB₂ comme décrit dans M. Rinaldi-Carmona et al., J. Pharmacol. Exp. Ther. 1996, 278, 871-878 et 1998, 284, 644-650.

La nature agoniste inverse des composés selon l'invention a été démontrée dans les modèles d'activation de l'adénylate-cyclase (stimulée par de la forskoline) comme décrit dans M Portier et al., J. Pharmacol. Exp. Ther. 1999, 288, 582-589.

Les composés selon l'invention possèdent également une bonne affinité *in vivo* pour les récepteurs CB₂ présents au niveau de la rate de souris lorsqu'ils sont administrés par voie orale. Les essais ont été réalisés selon les conditions expérimentales décrites par Rinaldi-Carmona et al., J. Pharmacol. Exp. Ther., 1998, 284, 644-650. L'affinité des composés est exprimée sous forme de DE50 (dose causant une inhibition de 50% de la liaison spécifique du ligand tritié utilisé ex vivo).

Les composés de la présente invention sont notamment des principes actifs compatibles avec leur utilisation en tant que médicaments et/ou compositions pharmaceutiques.

Selon un de ses aspects, la présente invention concerne l'utilisation d'un composé de formule (I) ou d'un de ses sels, pharmaceutiquement acceptable pour la préparation de médicaments destinés à prévenir ou à traiter toute pathologie humaine et/ou à usage vétérinaire. Ainsi les composés selon l'invention peuvent être utilisés chez l'homme ou chez l'animal (notamment chez les mammifères incluant de façon non limitative les chiens, les chats, les chevaux, les bovins, les moutons) pour la prévention ou le traitement de maladies impliquant les récepteurs CB₂.

On peut par exemple citer les maladies ou affections ci-après :
- Les désordres du système immunitaire : notamment les maladies auto-immunes incluant de façon non exhaustive : le psoriasis, le lupus érythémateux, les maladies du tissu conjonctif ou connectivités, le syndrome de Sjögrer's, la spondylarthrite ankylosante, la polyarthrite rhumatoïde, l'arthrite réactionnelle, la spondylarthrite indifférenciée, la maladie de Charcot, la maladie de Behcet's, les anémies auto-immunes hémolytiques, la sclérose en plaques, la sclérose latérale amyotrophique, les amyloses, le rejet de greffe, et les maladies affectant la lignée plasmocytaire ;
- Les maladies allergiques : notament l'hypersensibilité retardée ou immédiate, l'asthme, la rhinite allergique, la dermatite de contact et conjonctivite allergique ;
- Les maladies infectieuses parasitaire, virale ou bactérienne incluant notamment le SIDA et les méningites ;
- L'amylose et les maladies affectant les lignées du système lympho-hématopoïétique ;
- Les maladies chroniques du foie d'origine alcoolique, la cirrhose, virale et toxique ainsi que les stéatohépatites d'origine non alcoolique et le cancer primaire du foie ;
- Les maladies inflammatoires : notamment les maladies articulaires: l'arthrite, l'arthrite rhumatoïde, l'ostéoarthrite, la spondylite, la goutte, la vascularite, la maladie de Crohn, la maladie du colon irritable syndrome de colon irritable (en anglais IBD : inflammatory bowel disease et IBS : irritable bowel syndrome) et la colite ulcérative pancréatite aiguë ;
- Les maladies osseuses et de l'ostéoporose ;
- La douleur : notamment les douleurs chroniques de type inflammatoire, les douleurs neuropathiques et les douleurs aiguës périphériques ;
- Les affections oculaires : notamment l'hypertension oculaire et le glaucome ;
- Les affections pulmonaires : maladies des voies respiratoires, asthme, bronchite chronique, obstruction chronique des voies respiratoires (en anglais COPD : chronic obstructive pulmonary disease), emphysème ;
- Les maladies du système nerveux central et les maladies neuro dégénératives : notamment le syndrome de Tourette, la maladie de Parkinson, la maladie d'Alzheimer, la démence sénile, la chorée, la chorée de Huntington, l'épilepsie, les psychoses, la dépression et les lésions de la moelle épinière.

Le composé de formule (I) selon l'invention peut être utilisé comme médicament pour le traitement ou la prévention de :
- La migraine, du stress, des maladies d'origine psychosomatique, des crises de panique (attaque de panique ou crise d'angoisse aiguë), de l'épilepsie, des troubles du mouvement, les vertiges, les vomissements, les nausées en particulier ceux consécutifs à une chimiothérapie ;
- Les maladies cardiovasculaires en particulier hypertension, artériosclérose, crise cardiaque, ischémie cardiaque ;
- L'ischémie rénale ;
- Les cancers : notamment les tumeurs bénignes de la peau, papillomes et tumeurs cancéreuses, les tumeurs de la prostate, les tumeurs cérébrales (exemples : glioblastomes, médullo-épithéliomes, médulloblastomes, neuroblastomes, tumeurs d'origine embryonnaires, astrocytomes, astroblastomes, épendymomes, oligodendrogliomes, tumeur du plexus, neuro-épithéliomes, tumeur de l'épiphyse, épendymoblastomes, neuroectodermique, méningiomes malins, sarcomatoses, mélanomes malins, schwannomes) ;
- Les troubles gastro-intestinaux, les troubles diarrhéiques, les ulcères, les troubles vésicaux et urinaires, les néphrites, les troubles d'origine endocrinienne, le choc hémorragique, le choc septique, la maladie de Raynaud et les troubles de la fertilité ;
- L'obésité; le diabète de type II, le syndrome métabolique, la résistance à l'insuline et l'inflammation du tissu adipeux.

Plus particulièrement, les composés de formule (I) selon la présente invention seront utiles pour la préparation de médicaments permettant la prévention et/ou le traitement de la douleur, des maladies inflammatoires, des maladies auto-immunes, des maladies allergiques, des maladies infectieuses, des maladies neuro dégénératives, des maladies cardiovasculaires, des cancers, des maladies gastro-intestinales, de l'obésité, du diabète de type II, de la résistance à l'insuline et de l'inflammation du tissu adipeux.

L'utilisation des composés selon l'invention pour la préparation de médicaments destinés à la prévention et/ou le traitement des maladies mentionnées ci- dessus fait partie intégrante de l'invention.

Les composés de formule (I) ci-dessus, ou l'un de leurs sels pharmaceutiquement acceptables peuvent être utilisés à des doses journalières de 0,01 à 100 mg par kilo de poids corporel du mammifère à traiter, de préférence à des doses journalières de 0,1 à 50 mg/kg. Chez l'être humain, la dose peut varier de préférence de 0,1 à 4000 mg par jour, plus particulièrement de 0,5 à 1000 mg selon l'âge du sujet à traiter ou le type de traitement : prophylactique ou curatif.

Ainsi, selon un autre de ses aspects, la présente invention concerne des compositions pharmaceutiques renfermant, en tant que principe actif, un composé de formule (I), ou l'un de ses sels pharmaceutiquement acceptables ainsi qu'un ou plusieurs excipients pharmaceutiquement acceptables.

Dans les compositions pharmaceutiques de la présente invention pour l'administration par voie orale, sublinguale, inhalée, sous-cutanée, intramusculaire, intraveineuse, topique, locale, intratrachéale, intranasale, transdermique, locale ou rectale, les principes actifs peuvent être administrés sous formes unitaires d'administration, en mélange avec des supports pharmaceutiques classiques, aux animaux et aux êtres humains.

Les formes unitaires d'administration appropriées comprennent les formes par voie orale telles que les comprimés, les gélules molles ou dures, les poudres, les granules, les solutions ou suspensions orales, les formes d'administration sublinguale, buccale, intratrachéale, intraoculaire, intranasale, par inhalation, les aérosols, les formes d'administration topique, transdermique, les implants, les formes d'administration sous-cutanée, intramusculaire, intraveineuse et les formes d'administration rectale.

Pour l'administration topique on peut utiliser les composés selon l'invention dans des crèmes, des pommades, des gels, ou des lotions.

A titre d'exemple, une forme unitaire d'administration d'un composé selon l'invention sous forme de comprimé peut comprendre les composants suivants :

| | | |
|---|---|---|
| Composé selon l'invention | : | 50,0 mg |
| Mannitol | : | 223,75 mg |
| Croscarmellose sodique | : | 6,0 mg |
| Amidon de maïs | : | 15,0 mg |
| Hydroxypropyl-méthylcellulose | : | 2,25 mg |
| Stéarate de magnésium | : | 3,0 mg |

Par voie orale, la dose de principe actif administrée par jour peut atteindre 0,01 à 100 mg/kg, en une ou plusieurs prises, préférentiellement 0,02 à 50 mg/kg.

Il peut y avoir des cas particuliers où des dosages plus élevés ou plus faibles sont appropriés ; de tels dosages ne sortent pas du cadre de l'invention. Selon la pratique habituelle, le dosage approprié à chaque patient est déterminé par le médecin selon le mode d'administration, le poids et la réponse dudit patient.

La présente demande décrit une méthode de traitement des pathologies ci-dessus indiquées qui comprend l'administration, à un patient, d'une dose efficace d'un composé selon l'invention, ou d'un de ses sels pharmaceutiquement acceptables.

Les composés selon l'invention pourront également être utilisés pour la préparation de compositions à usage vétérinaire.

De plus, les composés selon l'invention, tels quels ou sous forme radiomarquée peuvent être utilisés comme outils pharmacologiques chez l'homme ou chez l'animal, pour la détection et le marquage des récepteurs aux cannabinoïdes CB₂.

## Revendications

1. Composé répondant à la formule (I) : 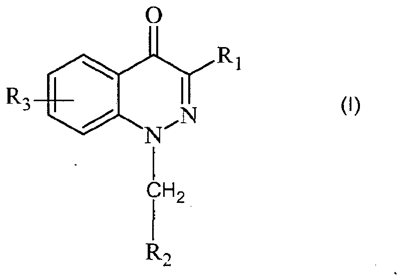 dans laquelle :
- R₁ représente :
. un phényle non substitué ou substitué une ou plusieurs fois par des substituants choisis indépendamment parmi un atome d'halogène, un groupe Alk, un groupe OAlk, un cyano, un groupe -NHSO₂Alk, un groupe -SO₂Alk ;
. un naphtyle non substitué ou substitué une ou plusieurs fois par des substituants choisis indépendamment parmi un atome d'halogène, un groupe Alk, un groupe OAlk;
. un pyridyle non substitué ou substitué une ou plusieurs fois par des substituants choisis indépendamment parmi un atome d'halogène, un groupe Alk, un groupe OAlk;
. un 1-benzothiényle non substitué ou substitué une ou plusieurs fois par des substituants choisis indépendamment parmi un atome d'halogène, un groupe Alk, un groupe OAlk ;
. un 1,3-benzodioxolyle non substitué ou substitué une ou plusieurs fois par des substituants choisis indépendamment parmi un atome d'halogène, un groupe Alk, un groupe OAlk ;
- R₂ représente un groupe aromatique ou hétéroaromatique, ledit groupe étant non substitué ou substitué une ou plusieurs fois par des substituants choisis indépendamment parmi un atome d'halogène, un groupe Alk, un groupe OAlk, un cyano ;
- R₃ représente un atome d'hydrogène, un atome d'halogène, un groupe Alk, un groupe OAlk ;
- Alk représente un (C₁-C₄)alkyle non substitué ou substitué une ou plusieurs fois par un atome de fluor ;
à l'état de base ou de sel d'addition à un acide.

2. Composé de formule (I) selon la revendication 1 dans laquelle :
- R₁ représente :
. un phényle non substitué ou substitué une ou deux fois par des substituants choisis indépendamment parmi un atome d'halogène, un groupe Alk, un groupe OAlk, un groupe -SO₂Alk ;
. un naphtyle ;
. un pyridyle substitué par un groupe Alk ou un groupe OAlk ;
. un 1-benzothiényle ;
. un 1,3-benzodioxolyle substitué par un ou plusieurs atomes de fluor ;
- R₂ représente :
. un phényle non substitué ou substitué une ou deux fois par des substituants choisis indépendamment parmi un atome d'halogène, un groupe Alk, un cyano ;
. un thiényle ou un pyridyle substitué par un atome d'halogène ou un groupe Alk ;
- R₃ représente un atome d'hydrogène, d'halogène ou un groupe Alk ;
- Alk représente un (C₁-C₄)alkyle non substitué ou substitué une ou plusieurs par un atome de fluor ;
à l'état de base ou de sel d'addition à un acide.

3. Composé de formule (I) selon la revendication 1 dans laquelle :
- R₁ représente :
. un phényle, un 4-chlorophényle, un 2-fluorophényle, un 3-fluorophényle, un 2-méthylphényle, un 4-méthylphényle, un 3-isopropylphényle, un 2-trifluorométhylphényle, un 3-trifluorométhylphényle, un 2-méthoxyphényle, un 4-méthoxyphényle, un 2-trifluorométhoxyphényle, un 3-trifluorométhoxyphényle, un 4-trifluorométhoxyphényle, un 2,3-dichlorophényle, un 3,5-dichlorophényle, un 2,3-difluorophényle, un 2,5-difluorophényle, un 3-chloro-2-fluorophényle, un 2-chloro-3-trifluorométhylphényle, un 3-chloro-5-trifluorométhylphényle, un 4-chloro-2-trifluorométhylphényle, un 3-bromo-2-fluorophényle, un 2-fluoro-3-méthylphényle, un 2-fluoro-4-méthylphényle, un 2-fluoro-3-trifluorométhylphényle, un 3-fluoro-2-trifluorométhylphényle, un 3-fluoro-5-trifluorométhylphényle, un 3-fluoro-2-méthoxyphényle, un 4-fluoro-2-méthoxyphényle, un 2-fluoro-5-trifluorométhoxyphényle, un 3-isopropyl-6-méthoxyphényle, un 3-*tert*-butyl-6-méthoxyphényle, un 2-(méthylsulfonyl)phényle ;
. un naphtyle ;
. un 2-méthoxypyrid-5-yle, un 2-trifluorométhylpyrid-3-yle ;
. un benzothièn-2-yle, un benzothièn-7-yle ;
. un 2,2-difluorobenzodioxol-4-yle;
- R₂ représente:
. un phényle, un 4-chlorophényle, un 2-fluorophényle, un 4-fluorophényle, un 2,4-dichlorophényle, un 2,4-difluorophényle, un 2-chloro-4-fluorophényle, un 4-chloro-2-fluorophényle, un 2-fluoro-4-méthylphényle, un 2-fluoro-4-trifluorométhylphényle, un 2-fluoro-4-cyanophényle ;
. un 2-chlorothièn-5-yle, un 3-trifluorométhylpyrid-6-yle, un 5-chloropyrid-2-yle, un 3,5-difluoropyrid-2-yle ;
- R₃ représente un atome d'hydrogène, un atome de chlore ou un méthoxy ;
à l'état de base ou de sel d'addition à un acide.

4. Composés de formule (I), selon la revendication 1 choisi parmi :
- 1-(2,4-Difluorobenzyl)-3-[2-fluoro-3-(trifluorométhyl)phényl]-8-méthoxycinnolin-4(1*H*)-one ;
- 3-[2-Chloro-3-(trifluorométhyl)phényl]-1-(2,4-difluorobenzyl)-8-méthoxycinnolin-4(1*H*)-one ;
- 3-[2-Chloro-3-(trifluorométhyl)phényl]-1-(2,4-difluorobenzyl)cinnolin-4(1*H*)-one ;
- 3-(2,3-Dichlorophényl)-1-(2,4-difluorobenzyl)-8-méthoxycinnolin-4(1*H*)-one ;
- 1-(2,4-Difluorobenzyl)-3-[2-fluoro-3-(trifluorométhyl)phényl]cinnolin-4(1*H*)-one ;
- 1-(2,4-Difluorobenzyl)-3-[3-fluoro-2-(trifluorométhyl)phényl]cinnolin-4(1*H*)-one ;
- 1-(4-Chloro-2-fluorobenzyl)-3-[2-fluoro-3-(trifluorométhyl)phényl]cinnolin-4(1*H*)-one ;
- 1-(2,4-Difluorobenzyl)-3-(3-isopropylphényl)cinnolin-4(1*H*)-one ;
- 1-(2,4-Difluorobenzyl)-3-[3-fluoro-2-(trifluorométhyl)phényl]-8-méthoxycinnolin-4(1*H*)-one ;
- 1-(2,4-Difluorobenzyl)-8-méthoxy-3-[3-(trifluorométhoxy)phényl]cinnolin-4(1*H*)-one ;
- 4-{[3-(2,3-Dichlorophényl)-4-oxocinnolin-1(4*H*)-yl]méthyl}-3-fluorobenzonitrile ;
- 3-Fluoro-4-({3-[2-fluoro-3-(trifluorométhyl)phényl]-4-oxocinnolin-1(4*H*)-yl}méthyl)benzonitrile ;
- 1-(2,4-Difluorobenzyl)-3-(4-fluoro-2-méthoxyphényl)cinnolin-4(1*H*)-one ;
- 3-(2,3-Dichlorophényl)-1-(2,4-difluorobenzyl)cinnolin-4(1*H*)-one ;
- 1-(2,4-Difluorobenzyl)-3-(5-fluoro-2-méthoxyphényl)cinnolin-4(1*H*)-one ;
- 1-(2,4-Difluorobenzyl)-3-(2-méthoxyphényl)cinnolin-4(1*H*)-one ;
- 3-(3-bromo-2-fluorophényl)-1-(2,4-difluorobenzyl)cinnolin-4(1*H*)-one
- 1-(2,4-Difluorobenzyl)-3-(2-fluoro-3-méthylphényl)cinnolin-4(1*H*)-one ;
- 1-(2,4-Difluorobenzyl)-3-[2-(trifluorométhyl)phényl]cinnolin-4(1*H*)-one ;
- 1-(2,4-Difluorobenzyl)-3-[2-(trifluorométhoxy)phényl]cinnolin-4(1*H*)-one ;
- 1-(2,4-Difluorobenzyl)-3-[3-(trifluorométhoxy)phényl]cinnolin-4(1*H*)-one ;
- 1-(2-Chloro-4-fluorobenzyl)-3-[2-fluoro-3-(trifluorométhyl)phényl]cinnolin-4(1*H*)-one ;
- 1-(4-Chloro-2-fluorobenzyl)-3-(5-fluoro-2-méthoxyphényl)cinnolin-4(1*H*)-one ;
- 3-(3-Chloro-2-fluorophényl)-1-(2,4-difluorobenzyl)cinnolin-4(1*H*)-one ;
- 3-Fluoro-4-({4-oxo-3-[2-(trifluorométhyl)phényl]cinnolin-1(4*H*)-yl}méthyl)benzonitrile ;
- 1-(2,4-Difluorobenzyl)-3-(4-méthylphényl)cinnolin-4(1*H*)-one ;
- 1-(2,4-Difluorobenzyl)-3-[3-(trifluorométhyl)phényl]cinnolin-4(1*H*)-one ;
- 1-[(5-Chloro-2-thiényl)méthyl]-3-[2-fluoro-3-(trifluorométhyl)phényl]cinnolin-4(1*H*)-one;
- 1-(2,4-Difluorobenzyl)-3-[2-(trifluorométhyl)pyridin-3-yl]cinnolin-4(1*H*)-one ;
- 3-(1-Benzothièn-7-yl)-1-(2,4-difluorobenzyl)cinnolin-4(1*H*)-one ;
- 1-Benzyl-3-(3-isopropylphényl)cinnolin-4(1*H*)-one ;
- 1-(2,4-Difluorobenzyl)-3-(5-isopropyl-2-méthoxyphényl)cinnolin-4(1*H*)-one ;
- 1-(2,4-Difluorobenzyl)-3-(1-naphthyl)cinnolin-4(1*H*)-one ;
- 1-(2,4-Difluorobenzyl)-3-[2-(trifluorométhyl)pyridin-3-yl]cinnolin-4(1*H*)-one ;
- 1-(2,4-Difluorobenzyl)-3-[2-fluoro-5-(trifluorométhoxy)phényl]cinnolin-4(1*H*)-one ;
- 1-(2,4-Difluorobenzyl)-3-(4-méthoxyphényl)cinnolin-4(1*H*)-one ;
- 3-(2,2-Difluoro-1,3-benzodioxol-4-yl)-1-(2,4-difluorobenzyl)cinnolin-4(1*H*)-one ;
- 3-(4-Chlorophényl)-1-(2,4-difluorobenzyl)cinnolin-4(1*H*)-one ;
- 1-[(5-Chloro-2-thiényl)méthyl]-3-(2,3-dichlorophényl)cinnolin-4(1*H*)-one ;
- 1-(4-Chloro-2-fluorobenzyl)-3-[3-(trifluorométhyl)phényl]cinnolin-4(1*H*)-one ;
- 1-(4-Fluorobenzyl)-3-(5-fluoro-2-méthoxyphényl)cinnolin-4(1*H*)-one ;
- 1-(2-Fluoro-4-méthylbenzyl)-3-[2-fluoro-3-(trifluorométhyl)phényl]cinnolin-4(1*H*)-one ;
- 1-(2-Fluorobenzyl)-3-(5-fluoro-2-méthoxyphényl)cinnolin-4(1*H*)-one ;
- 1-(2,4-Difluorobenzyl)-3-(2-fluoro-4-méthylphényl)cinnolin-4(1*H*)-one ;
- 1-(4-Fluorobenzyl)-3-[3-(trifluorométhyl)phényl]cinnolin-4(1*H*)-one ;
- 1-(2,4-Difluorobenzyl)-3-(3-fluorophényl)cinnolin-4(1*H*)-one ;
- 3-(5-Fluoro-2-méthoxyphényl)-1-[2-fluoro-4-(trifluorométhyl)benzyl]cinnolin-4(1*H*)-one ;
- 1-(4-Fluorobenzyl)-3-[2-fluoro-3-(trifluorométhyl)phényl]cinnolin-4(1*H*)-one ;
- 3-(5-Fluoro-2-méthoxyphényl)-1-(2-fluoro-4-méthylbenzyl)cinnolin-4(1*H*)-one ;
- 1-(2-Chloro-4-fluorobenzyl)-3-(5-fluoro-2-méthoxyphényl)cinnolin-4(1*H*)-one ;
- 1-(2,4-Difluorobenzyl)-3-(2,5-difluorophényl)cinnolin-4(1*H*)-one ;
- 1-(2,4-Difluorobenzyl)-3-[3-fluoro-5-(trifluorométhyl)phényl]cinnolin-4(1*H*)-one ;
- 1-(2,4-Difluorobenzyl)-3-(2-méthylphényl)cinnolin-4(1*H*)-one ;
- 1-(2,4-Difluorobenzyl)-3-(2-naphthyl)cinnolin-4(1*H*)-one ;
- 1-(2,4-Difluorobenzyl)-3-(2,3-difluorophényl)cinnolin-4(1*H*)-one ;
- 1-[2-Fluoro-4-(trifluorométhyl)benzyl]-3-[2-fluoro-3-(trifluorométhyl)phényl]cinnolin-4(1*H*)-one ;
- 3-[3-Chloro-5-(trifluorométhyl)phényl]-1-(2,4-difluorobenzyl)cinnolin-4(1*H*)-one ;
- 3-(3,5-Dichlorophenyl)-1-(2,4-difluorobenzyl)cinnolin-4(1*H*)-one;
- 1-(2,4-Difluorobenzyl)-3-(2-fluorophényl)cinnolin-4(1*H*)-one ;
- 1-(2,4-Dichlorobenzyl)-3-(5-fluoro-2-méthoxyphényl)cinnolin-4(1*H*)-one ;
- 3-[2-Fluoro-3-(trifluorométhyl)phényl]-1-{[5-(trifluorométhyl)pyridin-2-yl]méthyl}cinnolin-4(1*H*)-one ;
- 3-(5-*tert*-butyl-2-méthoxyphényl)-1-(2,4-difluorobenzyl)cinnolin-4(1*H*)-one ;
- 1-[2-Fluoro-4-(trifluorométhyl)benzyl]-3-[3-(trifluorométhyl)phényl]cinnolin-4(1*H*)-one;
- 3-[4-Chloro-2-(trifluorométhyl)phényl]-1-(2,4-difluorobenzyl)cinnolin-4(1*H*)-one ;
- 1-(2,4-Fifluorobenzyl)-3-(5-fluoro-2-hydroxyphényl)cinnolin-4(1*H*)-one ;
- 1-(2,4-Difluorobenzyl)-3-phénylcinnolin-4(1*H*)-one ;
- 1-(2,4-Dichlorobenzyl)-3-[3-(trifluorométhyl)phényl]cinnolin-4(1*H*)-one ;
- 1-(2-Chloro-4-fluorobenzyl)-3-[3-(trifluorométhyl)phényl]cinnolin-4(1*H*)-one ;
- 1-(2-Fluoro-4-methylbenzyl)-3-[3-(trifluorométhyl)phényl]cinnolin-4(1*H*)-one ;
- 3-(1-Benzothien-2-yl)-1-(4-chlorobenzyl)cinnolin-4(1*H*)-one ;
- 1-(2-Fluorobenzyl)-3-[2-fluoro-3-(trifluorométhyl)phényl]cinnolin-4(1*H*)-one ;
- 1-[(5-Chloro-2-thiényl)méthyl]-3-(5-fluoro-2-méthoxyphényl)cinnolin-4(1*H*)-one ;
- 1-(2,4-Difluorobenzyl)-3-[4-(trifluorométhoxy)phényl]cinnolin-4(1*H*)-one ;
- 1-(2,4-Difluorobenzyl)-3-(6-méthoxypyridin-3-yl)cinnolin-4(1*H*)-one;
- 3-(2,3-Dichlorophényl)-1-{[5-(trifluorométhyl)pyridin-2-yl]méthyl}cinnolin-4(1*H*)-one ;
- 7-Chloro-1-(2,4-difluorobenzyl)-3-[2-fluoro-3-(trifluorométhyl)phényl]cinnolin-4(1*H*)-one ;
- 1-(2,4-Difluorobenzyl)-3-[3-fluoro-2-(trifluorométhyl)phényl]-7-méthoxycinnolin-4(1*H*)-one ;
- 7-Chloro-3-[2-chloro-3-(trifluorométhyl)phényl]-1-(2,4-difluorobenzyl)cinnolin-4(1*H*)-one ;
- 1-(2,4-Difluorobenzyl)-7-méthoxy-3-[3-(trifluorométhoxy)phényl]cinnolin-4(1*H*)-one ;
- 4-({3-[2-Chloro-3-(trifluorométhyl)phényl]-4-oxocinnolin-1(4*H*)-yl}méthyl)-3-fluorobenzonitrile ;
- 7-Chloro-1-(2,4-difluorobenzyl)-3-[3-fluoro-2-(trifluorométhyl)phényl]cinnolin-4(1*H*)-one ;
- 7-Chloro-3-(2,3-dichlorophényl)-1-(2,4-difluorobenzyl)cinnolin-4(1*H*)-one ;
- 7-Chloro-1-(2,4-difluorobenzyl)-3-[3-(trifluorométhoxy)phényl]cinnolin-4(1*H*)-one ;
- 7-Chloro-1-(2,4-difluorobenzyl)-3-[2-(méthylsulfonyl)phényl]cinnolin-4(1*H*)-one ;
- 1-[(5-Chloropyridin-2-yl)méthyl]-3-[2-fluoro-3-(trifluorométhyl)phényl]cinnolin-4(1*H*)-one ;
- 6-Chloro-3-[2-chloro-3-(trifluorométhyl)phényl]-1-(2,4-difluorobenzyl)cinnolin-4(1*H*)-one ;
- 6-Chloro-1-(2,4-difluorobenzyl)-3-[2-fluoro-3-(trifluorométhyl)phényl]cinnolin-4(1*H*)-one ;
- 1-(2,4-Difluorobenzyl)-3-[2-fluoro-3-(trifluorométhyl)phényl]-6-méthoxycinnolin-4(1*H*)-one ;
- 6-Chloro-1-[(3,5-difluoropyridin-2-yl)méthyl]-3-[2-fluoro-3-(trifluorométhyl)phényl]cinnolin-4(1*H*)-one ;
à l'état de base ou de sel d'addition à un acide.

5. Procédé de préparation des composés de formule (I) selon l'une quelconque des revendications 1 à 4, **caractérisé en ce que** :
On fait réagir en présence d'une base, un composé de formule : 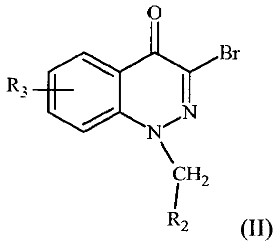 dans laquelle R₂ et R₃ sont tels que définis pour un composé de formule (I) à la revendication 1, avec un composé de formule :
R₁-B(OH)₂ (III)
dans laquelle R₁ est tel que défini pour un composé de formule (I) à la revendication 1.

6. Médicament, **caractérisé en ce qu'**il contient un composé de formule (I) selon l'une quelconque des revendications 1 à 4, ou encore un sel pharmaceutiquement acceptable du composé de formule (I).

7. Composition pharmaceutique, **caractérisé en ce qu'**elle comprend un composé de formule (I) selon l'une quelconque des revendications 1 à 4, un sel pharmaceutiquement acceptable de ce composé, ainsi qu'au moins un excipient pharmaceutiquement acceptable.

8. Utilisation d'un composé de formule (I) selon l'une quelconque des revendications 1 à 4 pour la préparation d'un médicament destiné à traiter et à prévenir toute pathologie dans laquelle les récepteurs aux cannabinoïdes CB₂ sont impliqués.

9. Utilisation selon la revendication 8 pour le traitement et la prévention de la douleur, des maladies inflammatoires, des maladies auto-immunes, des maladies allergiques, des maladies infectieuses, des maladies neurogénératives, des maladies cardiovasculaires, des cancers, des maladies gastro-intestinales, de l'obésité, du diabète de type II, de la résistance à l'insuline et l'inflammation du tissu adipeux.

## Patentansprüche

1. Verbindung der Formel (I): 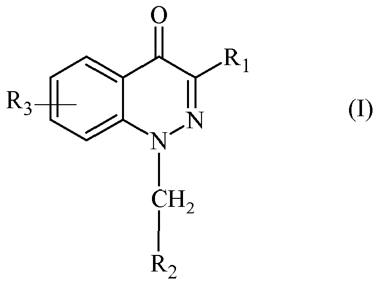 worin:
R₁ für:
. ein Phenyl, das unsubstituiert oder ein- oder mehrfach durch Substituenten, die unabhängig voneinander aus einem Halogenatom, einer Alk-Gruppe, einer OAlk-Gruppe, einem Cyano, einer -NHSO₂Alk-Gruppe und einer -SO₂Alk-Gruppe aus-gewählt sind, substituiert ist;
. ein Naphthyl, das unsubstituiert oder ein- oder mehrfach durch Substituenten, die unabhängig voneinander aus einem Halogenatom, einer Alk-Gruppe und einer OAlk-Gruppe ausgewählt sind, substituiert ist;
. ein Pyridyl, das unsubstituiert oder ein- oder mehrfach durch Substituenten, die unabhängig voneinander aus einem Halogenatom, einer Alk-Gruppe und einer OAlk-Gruppe ausgewählt sind, substituiert ist;
. ein 1-Benzothienyl, das unsubstituiert oder ein-oder mehrfach durch Substituenten, die unabhängig voneinander aus einem Halogenatom, einer Alk-Gruppe und einer OAlk-Gruppe ausgewählt sind, substituiert ist;
. ein 1,3-Benzodioxolyl, das unsubstituiert oder ein- oder mehrfach durch Substituenten, die unabhängig voneinander aus einem Halogenatom, einer Alk-Gruppe und einer OAlk-Gruppe ausgewählt sind, substituiert ist; steht;
- R₂ für eine aromatische oder heteroaromatische Gruppe steht, wobei die Gruppe unsubstituiert oder ein- oder mehrfach durch Substituenten, die unabhängig voneinander aus einem Halogenatom, einer Alk-Gruppe, einer OAlk-Gruppe und einem Cyano ausgewählt sind, substituiert ist;
- R₃ für ein Wasserstoffatom, ein Halogenatom, eine Alk-Gruppe oder eine OAlk-Gruppe steht;
- Alk für ein (C₁-C₄)-Alkyl, das unsubstituiert oder ein- oder mehrfach durch ein Fluoratom substituiert ist, steht;
in Basen- oder Säureadditionssalzform.

2. Verbindung der Formel (I) nach Anspruch 1, worin:
R₁ für:
. ein Phenyl, das unsubstituiert oder ein- oder zweifach durch Substituenten, die unabhängig voneinander aus einem Halogenatom, einer Alk-Gruppe, einer OAlk-Gruppe und einer -SO₂Alk-Gruppe ausgewählt sind, substituiert ist;
. ein Naphthyl;
. ein Pyridyl, das durch eine Alk-Gruppe oder eine OAlk-Gruppe substituiert ist;
. ein 1-Benzothienyl;
. ein 1,3-Benzodioxolyl, das durch ein oder mehrere Fluoratome substituiert ist;
steht;
- R₂ für:
. ein Phenyl, das unsubstituiert oder ein- oder zweifach durch Substituenten, die unabhängig voneinander aus einem Halogenatom, einer Alk-Gruppe und einem Cyano ausgewählt sind, substituiert ist;
. ein Thienyl oder ein Pyridyl, das durch ein Halogenatom oder eine Alk-Gruppe substituiert ist;
steht;
- R₃ für ein Wasserstoffatom, ein Halogenatom oder eine OAlk-Gruppe steht;
- Alk für ein (C₁-C₄)-Alkyl, das unsubstituiert oder ein- oder mehrfach durch ein Fluoratom substituiert ist, steht;
in Basen- oder Säureadditionssalzform.

3. Verbindung der Formel (I) nach Anspruch 1, worin:
R₁ für:
. ein Phenyl, ein 4-Chlorphenyl, ein 2-Fluorphenyl, ein 3-Fluorphenyl, ein 2-Methylphenyl, ein 4-Methylphenyl, ein 3-Isopropylphenyl, ein 2-Trifluormethylphenyl, ein 3-Trifluormethylphenyl, ein 2-Methoxyphenyl, ein 4-Methoxyphenyl, ein 2-Trifluormethoxyphenyl, ein 3-Trifluormethoxyphenyl, ein 4-Trifluormethoxyphenyl, ein 2,3-Dichlorphenyl, ein 3,5-Dichlorphenyl, ein 2,3-Difluorphenyl, ein 2,5-Difluorphenyl, ein 3-Chlor-2-fluorphenyl, ein 2-Chlor-3-trifluormethylphenyl, ein 3-Chlor-5-trifluormethylphenyl, ein 4-Chlor-2-trifluormethylphenyl, ein 3-Brom-2-fluorphenyl, ein 2-Fluor-3-methylphenyl, ein 2-Fluor-4-methylphenyl, ein 2-Fluor-3-trifluormethylphenyl, ein 3-Fluor-2-trifluormethylphenyl, ein 3-Fluor-5-trifluormethylphenyl, ein 3-Fluor-2-methoxyphenyl, ein 4-Fluor-2-methoxyphenyl, ein 2-Fluor-5-trifluormethoxyphenyl, ein 3-Isopropyl-6-methoxy-phenyl, ein 3-(*tert*-Butyl)-6-methoxyphenyl, ein 2-(Methylsulfonyl)phenyl;
. ein Naphthyl;
. ein 2-Methoxypyrid-5-yl, ein 2-Trifluormethyl-pyrid-3-yl;
. ein Benzothien-2-yl, ein Benzothien-7-yl;
. ein 2,2-Difluorbenzodioxol-4-yl
steht;
- R₂ für:
. ein Phenyl, ein 4-Chlorphenyl, ein 2-Fluorphenyl, ein 4-Fluorphenyl, ein 2,4-Dichlorphenyl, ein 2,4-Difluorphenyl, ein 2-Chlor-4-fluorphenyl, ein 4-Chlor-2-fluorphenyl, ein 2-Fluor-4-methylphenyl, ein 2-Fluor-4-trifluormethylphenyl, ein 2-Fluor-4-cyanophenyl;
. ein 2-Chlorthien-5-yl, ein 3-Trifluormethyl-pyrid-6-yl, ein 5-Chlorpyrid-2-yl, ein 3,5-Difluorpyrid-2-yl
steht;
- R₃ für ein Wasserstoffatom, ein Chloratom oder ein Methoxy steht;
in Basen- oder Säureadditionssalzform.

4. Verbindung der Formel (I) nach Anspruch 1, ausgewählt aus:
- 1-(2,4-Difluorbenzyl)-3-[2-fluor-3-(trifluormethyl)phenyl]-8-methoxycinnolin-4(1H)-on;
- 3-[2-Chlor-3-(trifluormethyl)phenyl]-1-(2,4-difluorbenzyl)-8-methoxycinnolin-4(1*H*)-on;
- 3-[2-Chlor-3-(trifluormethyl)phenyl]-1-(2,4-difluorbenzyl)cinnolin-4(1*H*)-on;
- 3-(2,3-Dichlorphenyl)-1-(2,4-difluorbenzyl)-8-methoxycinnolin-4(1*H*)-on;
- 1-(2,4-Difluorbenzyl)-3-[2-fluor-3-(trifluormethyl)phenyl]cinnolin-4(1*H*)-on;
- 1-(2,4-Difluorbenzyl)-3-[3-fluor-2-(trifluormethyl)phenyl]cinnolin-4(1*H*)-on;
- 1-(4-Chlor-2-fluorbenzyl)-3-[2-fluor-3-(trifluormethyl)phenyl]cinnolin-4(1*H*)-on;
- 1-(2,4-Difluorbenzyl)-3-(3-isopropylphenyl)cinnolin-4(1*H*)-on;
- 1-(2,4-Difluorbenzyl)-3-[3-fluor-2-(trifluormethyl)phenyl]-8-methoxycinnolin-4(1*H*)-on;
- 1-(2,4-Difluorbenzyl)-8-methoxy-3-[3-(trifluormethoxy)phenyl]cinnolin-4(1*H*)-on;
- 4-{[3-(2,3-Dichlorphenyl)-4-oxocinnolin-1(4*H*)-yl]methyl}-3-fluorbenzonitril;
- 3-Fluor-4-({3-[2-fluor-3-(trifluormethyl)phenyl]-4-oxocinnolin-1(4*H*)-yl}methyl)benzonitril;
- 1-(2,4-Difluorbenzyl)-3-(4-fluor-2-methoxyphenyl)cinnolin-4(1*H*)-on;
- 3-(2,3-Dichlorphenyl)-1-(2,4-difluorbenzyl)cinnolin-4(1*H*)-on;
- 1-(2,4-Difluorbenzyl)-3-(5-fluor-2-methoxyphenyl)cinnolin-4(1*H*)-on;
- 1-(2,4-Difluorbenzyl)-3-(2-methoxyphenyl)cinnolin-4(1*H*)-on;
- 3-(3-Brom-2-fluorphenyl)-1-(2,4-difluorbenzyl)cinnolin-4(1*H*)-on;
- 1-(2,4-Difluorbenzyl)-3-(2-fluor-3-methylphenyl)cinnolin-4(1*H*)-on;
- 1-(2,4-Difluorbenzyl)-3-[2-(trifluormethyl)phenyl]cinnolin-4(1*H*)-on;
- 1-(2,4-Difluorbenzyl)-3-[2-(trifluormethoxy)phenyl]cinnolin-4(1*H*)-on;
- 1-(2,4-Difluorbenzyl)-3-[3-(trifluormethoxy)phenyl]cinnolin-4(1*H*)-on;
- 1-(2-Chlor-4-fluorbenzyl)-3-[2-fluor-3-(trifluormethyl)phenyl]cinnolin-4(1*H*)-on;
- 1-(4-Chlor-2-fluorbenzyl)-3-(5-fluor-2-methoxyphenyl)cinnolin-4(1*H*)-on;
- 3-(3-Chlor-2-fluorphenyl)-1-(2,4-difluorbenzyl)cinnolin-4(1*H*)-on;
- 3-Fluor-4-({4-oxo-3-[2-(trifluormethyl)phenyl]cinnolin-1(4*H*)-yl}methyl)benzonitril;
- 1-(2,4-Difluorbenzyl)-3-(4-methylphenyl)cinnolin-4(1*H*)-on;
- 1-(2,4-Difluorbenzyl)-3-[3-(trifluormethyl)phenyl]cinnolin-4(1*H*)-on;
- 1-[(5-Chlor-2-thienyl)methyl]-3-[2-fluor-3-(trifluormethyl)phenyl]cinnolin-4(1*H*)-on;
- 1-(2,4-Difluorbenzyl)-3-[2-(trifluormethyl)pyridin-3-yl]cinnolin-4(1*H*)-on;
- 3-(1-Benzothien-7-yl)-1-(2,4-difluorbenzyl)cinnolin-4(1*H*)-on;
- 1-Benzyl-3-(3-isopropylphenyl)cinnolin-4(1*H*)-on;
- 1-(2,4-Difluorbenzyl)-3-(5-isopropyl-2-methoxyphenyl)cinnolin-4(1*H*)-on;
- 1-(2,4-Difluorbenzyl)-3-(1-naphthyl)cinnolin-4(1*H*)-on;
- 1-(2,4-Difluorbenzyl)-3-[2-(trifluormethyl)pyridin-3-yl]cinnolin-4(1*H*)-on;
- 1-(2,4-Difluorbenzyl)-3-[2-fluor-5-(trifluormethoxy)phenyl]cinnolin-4(1*H*)-on;
- 1-(2,4-Difluorbenzyl)-3-(4-methoxyphenyl)cinnolin-4(1*H*)-on;
- 3-(2,2-Difluor-1,3-benzodioxol-4-yl)-1-(2,4-difluorbenzyl)cinnolin-4(1*H*)-on;
- 3-(4-Chlorphenyl)-1-(2,4-difluorbenzyl)cinnolin-4(1*H*)-on;
- 1-[(5-Chlor-2-thienyl)methyl]-3-(2,3-dichlorphenyl)cinnolin-4(1*H*)-on;
- 1-(4-Chlor-2-fluorbenzyl)-3-[3-(trifluormethyl)phenyl]cinnolin-4(1*H*)-on;
- 1-(4-Fluorbenzyl)-3-(5-fluor-2-methoxyphenyl)cinnolin-4(1*H*)-on;
- 1-(2-Fluor-4-methylbenzyl)-3-[2-fluor-3-(trifluormethyl)phenyl]cinnolin-4(1*H*)-on;
- 1-(2-Fluorbenzyl)-3-(5-fluor-2-methoxyphenyl)cinnolin-4(1*H*)-on;
- 1-(2,4-Difluorbenzyl)-3-(2-fluor-4-methylphenyl)cinnolin-4(1*H*)-on;
- 1-(4-Fluorbenzyl)-3-[3-(trifluormethyl)phenyl]cinnolin-4(1*H*)-on;
- 1-(2,4-Difluorbenzyl)-3-(3-fluorphenyl)cinnolin-4(1*H*)-on;
- 3-(5-Fluor-2-methoxyphenyl)-1-[2-fluor-4-(trifluormethyl)benzyl]cinnolin-4(1*H*)-on;
- 1-(4-Fluorbenzyl)-3-[2-fluor-3-(trifluormethyl)phenyl]cinnolin-4(1*H*)-on;
- 3-(5-Fluor-2-methoxyphenyl)-1-(2-fluor-4-methylbenzyl)cinnolin-4(1*H*)-on;
- 1-(2-Chlor-4-fluorbenzyl)-3-(5-fluor-2-methoxyphenyl)cinnolin-4(1*H*)-on;
- 1-(2,4-Difluorbenzyl)-3-(2,5-difluorphenyl)cinnolin-4(1*H*)-on;
- 1-(2,4-Difluorbenzyl)-3-[3-fluor-5-(trifluormethyl)phenyl]cinnolin-4(1*H*)-on;
- 1-(2,4-Difluorbenzyl)-3-(2-methylphenyl)cinnolin-4(1*H*)-on;
- 1-(2,4-Difluorbenzyl)-3-(2-naphthyl)cinnolin-4(1*H*)-on;
- 1-(2,4-Difluorbenzyl)-3-(2,3-difluorphenyl)cinnolin-4(1*H*)-on;
- 1-[2-Fluor-4-(trifluormethyl)benzyl]-3-[2-fluor-3-(trifluormethyl)phenyl]cinnolin-4(1*H*)-on;
- 3-[3-Chlor-5-(trifluormethyl)phenyl]-1-(2,4-difluorbenzyl)cinnolin-4(1*H*)-on;
- 3-(3,5-Dichlorphenyl)-1-(2,4-difluorbenzyl)cinnolin-4(1*H*)-on;
- 1-(2,4-Difluorbenzyl)-3-(2-fluorphenyl)cinnolin-4(1*H*)-on;
- 1-(2,4-Dichlorbenzyl)-3-(5-fluor-2-methoxyphenyl)cinnolin-4(1*H*)-on;
- 3-[2-Fluor-3-(trifluormethyl)phenyl]-1-{[5-(trifluormethyl)pyridin-2-yl]methyl}cinnolin-4(1*H*)-on;
- 3-(5-(tert-Butyl)-2-methoxyphenyl)-1-(2,4-difluorbenzyl)cinnolin-4(1*H*)-on;
- 1-[2-Fluor-4-(trifluormethyl)benzyl]-3-[3-(trifluormethyl)phenyl]cinnolin-4(1*H*)-on;
- 3-[4-Chlor-2-(trifluormethyl)phenyl]-1-(2,4-difluorbenzyl)cinnolin-4(1*H*)-on;
- 1-(2,4-Difluorbenzyl)-3-(5-fluor-2-hydroxyphenyl)cinnolin-4(1*H*)-on;
- 1-(2,4-Difluorbenzyl)-3-phenylcinnolin-4(1*H*)-on;
- 1-(2,4-Dichlorbenzyl)-3-[3-(trifluormethyl)phenyl]cinnolin-4(1*H*)-on;
- 1-(2-Chlor-4-fluorbenzyl)-3-[3-(trifluormethyl)phenyl]cinnolin-4(1*H*)-on;
- 1-(2-Fluor-4-methylbenzyl)-3-[3-(trifluormethyl)phenyl]cinnolin-4(1*H*)-on;
- 3-(1-Benzothien-2-yl)-1-(4-chlorbenzyl)cinnolin-4(1*H*)-on;
- 1-(2-Fluorbenzyl)-3-[2-fluor-3-(trifluormethyl)phenyl]cinnolin-4(1*H*)-on;
- 1-[(5-Chlor-2-thienyl)methyl]-3-(5-fluor-2-methoxyphenyl)cinnolin-4(1*H*)-on;
- 1-(2,4-Difluorbenzyl)-3-[4-(trifluormethoxy)phenyl]cinnolin-4(1*H*)-on;
- 1-(2,4-Difluorbenzyl)-3-(6-methoxypyridin-3-yl)cinnolin-4(1*H*)-on;
- 3-(2,3-Dichlorphenyl)-1-{[5-(trifluormethyl)pyridin-2-yl]methyl}cinnolin-4(1*H*)-on;
- 7-Chlor-1-(2,4-difluorbenzyl)-3-[2-fluor-3-(trifluormethyl)phenyl]cinnolin-4(1*H*)-on;
- 1-(2,4-Difluorbenzyl)-3-[3-fluor-2-(trifluormethyl)phenyl]-7-methoxycinnolin-4(1H)-on;
- 7-Chlor-3-[2-chlor-3-(trifluormethyl)phenyl]-1-(2,4-difluorbenzyl)cinnolin-4(1*H*)-on;
- 1-(2,4-Difluorbenzyl)-7-methoxy-3-[3-(trifluormethoxy)phenyl]cinnolin-4(1*H*)-on;
- 4-({3-[2-Chlor-3-(trifluormethyl)phenyl]-4-oxocinnolin-1(4*H*)-yl}methyl)-3-fluorbenzonitril;
- 7-Chlor-1-(2,4-difluorbenzyl)-3-[3-fluor-2-(trifluormethyl)phenyl]cinnolin-4(1*H*)-on;
- 7-Chlor-3-(2,3-dichlorphenyl)-1-(2,4-difluorbenzyl)cinnolin-4(1*H*)-on;
- 7-Chlor-1-(2,4-difluorbenzyl)-3-[3-(trifluormethoxy)phenyl]cinnolin-4(1*H*)-on;
- 7-Chlor-1-(2,4-difluorbenzyl)-3-[2-(methylsulfonyl)phenyl]cinnolin-4(1*H*)-on;
- 1-[(5-Chlorpyridin-2-yl)methyl]-3-[2-fluor-3-(trifluormethyl)phenyl]cinnolin-4(1*H*)-on;
- 6-Chlor-3-[2-chlor-3-(trifluormethyl)phenyl]-1-(2,4-difluorbenzyl)cinnolin-4(1*H*)-on;
- 6-Chlor-1-(2,4-difluorbenzyl)-3-[2-fluor-3-(trifluormethyl)phenyl]cinnolin-4(1*H*)-on;
- 1-(2,4-Difluorbenzyl)-3-[2-fluor-3-(trifluormethyl)phenyl]-6-methoxycinnolin-4(1H)-on;
- 6-Chlor-1-[(3,5-difluorpyridin-2-yl)methyl]-3-[2-fluor-3-(trifluormethyl)phenyl]cinnolin-4(1*H*)-on;
in Basen- oder Säureadditionssalzform.

5. Verfahren zur Herstellung der Verbindungen der Formel (I) nach einem der Ansprüche 1 bis 4, **dadurch gekennzeichnet, dass**:
man eine Verbindung der Formel: 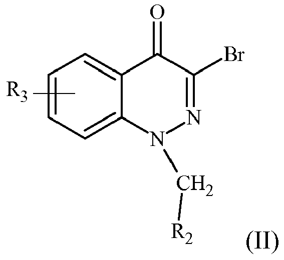 worin R₂ und R₃ wie für eine Verbindung der Formel (I) in Anspruch 1 definiert sind, in Gegenwart einer Base mit einer Verbindung der Formel:
R₁-B(OH)₂ (III)
worin R₁ wie für eine Verbindung der Formel (I) in Anspruch 1 definiert ist, umsetzt.

6. Medikament, **dadurch gekennzeichnet, dass** es eine Verbindung der Formel (I) nach einem der Ansprüche 1 bis 4 oder ein pharmazeutisch unbedenkliches Salz der Verbindung der Formel (I) enthält.

7. Pharmazeutische Zusammensetzung, **dadurch gekennzeichnet, dass** sie eine Verbindung der Formel (I) nach einem der Ansprüche 1 bis 4, ein pharmazeutisch unbedenkliches Salz dieser Verbindung sowie mindestens einen pharmazeutisch unbedenklichen Hilfsstoff umfasst.

8. Verwendung einer Verbindung der Formel (I) nach einem der Ansprüche 1 bis 4 zur Herstellung eines Medikaments zur Behandlung und Prävention jeder Pathologie, an denen CB₂-Cannabinoidrezeptoren beteiligt sind.

9. Verwendung nach Anspruch 8 zur Behandlung und Prävention von Schmerzen, entzündlichen Erkrankungen, Autoimmunerkrankungen, allergischen Erkrankungen, Infektionserkrankungen, neurodegenerativen Erkrankungen, Herz-Kreislauf-Erkrankungen, Krebserkrankungen, Magen-Darm-Erkrankungen, Obesitas, Typ-II-Diabetes, Insulinresistenz und Fettgewebeentzündung.

## Claims

1. Compound corresponding to the formula (I): 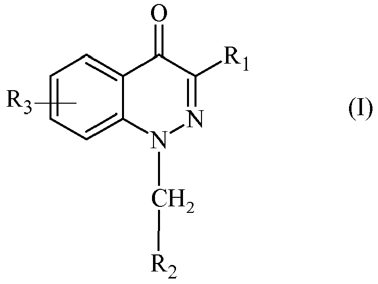 in which:
- R₁ represents:
. a phenyl which is unsubstituted or substituted one or more times by substituents chosen independently from a halogen atom, an Alk group, an OAlk group, a cyano, an -NHSO₂Alk group or an -SO₂Alk group;
. a naphthyl which is unsubstituted or substituted one or more times by substituents chosen independently from a halogen atom, an Alk group or an OAlk group;
. a pyridyl which is unsubstituted or substituted one or more times by substituents chosen independently from a halogen atom, an Alk group or an OAlk group;
. a 1-benzothienyl which is unsubstituted or substituted one or more times by substituents chosen independently from a halogen atom, an Alk group or an OAlk group;
. a 1,3-benzodioxolyl which is unsubstituted or substituted one or more times by substituents chosen independently from a halogen atom, an Alk group or an OAlk group;
- R₂ represents an aromatic or heteroaromatic group, the said group being unsubstituted or substituted one or more times by substituents chosen independently from a halogen atom, an Alk group, an OAlk group or a cyano;
- R₃ represents a hydrogen atom, a halogen atom, an Alk group or an OAlk group;
- Alk represents a (C₁-C₄)alkyl which is unsubstituted or substituted one or more times by a fluorine atom;
in the form of the base or of an addition salt with an acid.

2. Compound of formula (I) according to Claim 1, in which:
- R₁ represents:
a phenyl which is unsubstituted or substituted once or twice by substituents chosen independently from a halogen atom, an Alk group, an OAlk group or an -SO₂Alk group;
. a naphthyl;
. a pyridyl substituted by an Alk group or an OAlk group;
. a 1-benzothienyl;
. a 1,3-benzodioxolyl substituted by one or more fluorine atoms;
- R₂ represents:
. a phenyl which is unsubstituted or substituted once or twice by substituents chosen independently from a halogen atom, an Alk group or a cyano;
. a thienyl or a pyridyl which is substituted by a halogen atom or an Alk group;
- R₃ represents a hydrogen or halogen atom or an OAlk group;
- Alk represents a (C₁-C₄)alkyl which is unsubstituted or substituted one or more times by a fluorine atom;
in the form of the base or of an addition salt with an acid.

3. Compound of formula (I) according to Claim 1, in which:
- R₁ represents:
. a phenyl, a 4-chlorophenyl, a 2-fluorophenyl, a 3-fluorophenyl, a 2-methylphenyl, a 4-methylphenyl, a 3-isopropylphenyl, a 2-trifluoromethylphenyl, a 3-trifluoromethylphenyl, a 2-methoxyphenyl, a 4-methoxyphenyl, a 2-trifluoromethoxyphenyl, a 3-trifluoromethoxyphenyl, a 4-trifluoromethoxyphenyl, a 2,3-dichlorophenyl, a 3,5-dichlorophenyl, a 2,3-difluorophenyl, a 2,5-difluorophenyl, a 3-chloro-2-fluorophenyl, a 2-chloro-3-trifluoromethylphenyl, a 3-chloro-5-trifluoromethylphenyl, a 4-chloro-2-trifluoromethylphenyl, a 3-bromo-2-fluorophenyl, a 2-fluoro-3-methylphenyl, a 2-fluoro-4-methylphenyl, a 2-fluoro-3-trifluoromethylphenyl, a 3-fluoro-2-trifluoromethylphenyl, a 3-fluoro-5-trifluoromethylphenyl, a 3-fluoro-2-methoxyphenyl, a 4-fluoro-2-methoxyphenyl, a 2-fluoro-5-trifluoromethoxyphenyl, a 3-isopropyl-6-methoxyphenyl, a 3-(tert-butyl)-6-methoxyphenyl, a 2-(methylsulphonyl)phenyl;
. a naphthyl;
. a 2-methoxypyrid-5-yl, a 2-trifluoromethylpyrid-3-yl;
. a benzothien-2-yl, a benzothien-7-yl;
. a 2,2-difluorobenzodioxol-4-yl;
- R₂ represents:
. a phenyl, a 4-chlorophenyl, a 2-fluorophenyl, a 4-fluorophenyl, a 2,4-dichlorophenyl, a 2,4-difluorophenyl, a 2-chloro-4-fluorophenyl, a 4-chloro-2- fluorophenyl, a 2-fluoro-4-methylphenyl, a 2-fluoro-4-trifluoromethylphenyl or a 2-fluoro-4-cyanophenyl;
. a 2-chlorothien-5-yl, a 3-trifluoromethylpyrid-6-yl, a 5-chloropyrid-2-yl or a 3,5-difluoropyrid-2-yl;
- R₃ represents a hydrogen atom, a chlorine atom or a methoxy;
in the form of the base or of an addition salt with an acid.

4. Compound of formula (I) according to Claim 1, chosen from:
- 1-(2,4-difluorobenzyl)-3-[2-fluoro-3-(trifluoromethyl)phenyl]-8-methoxycinnolin-4(1*H*)-one;
- 3-[2-chloro-3-(trifluoromethyl)phenyl]-1-(2,4-difluorobenzyl)-8-methoxycinnolin-4(1*H*)-one;
- 3-[2-chloro-3-(trifluoromethyl)phenyl]-1-(2,4-difluorobenzyl)cinnolin-4(1*H*)-one;
- 3-(2,3-dichlorophenyl)-1-(2,4-difluorobenzyl)-8-methoxycinnolin-4(1*H*)-one;
- 1-(2,4-difluorobenzyl)-3-[2-fluoro-3-(trifluoromethyl)phenyl]cinnolin-4(1*H*)-one;
- 1-(2,4-difluorobenzyl)-3-[3-fluoro-2-(trifluoromethyl)phenyl]cinnolin-4(1*H*)-one;
- 1-(4-chloro-2-fluorobenzyl)-3-[2-fluoro-3-(trifluoromethyl)phenyl]cinnolin-4(1*H*)-one;
- 1-(2,4-difluorobenzyl)-3-(3-isopropylphenyl)cinnolin-4(1*H*)-one;
- 1-(2,4-difluorobenzyl)-3-[3-fluoro-2-(trifluoromethyl)phenyl]-8-methoxycinnolin-4(1*H*)-one;
- 1-(2,4-difluorobenzyl)-8-methoxy-3-[3-(trifluoromethoxy)phenyl]cinnolin-4(1*H*)-one;
- 4-{[3-(2,3-dichlorophenyl)-4-oxocinnolin-1(4*H*)-yl]methyl}-3-fluorobenzonitrile;
- 3-fluoro-4-({3-[2-fluoro-3-(trifluoromethyl)phenyl]-4-oxocinnolin-1(4H)-yl}methyl)benzonitrile;
- 1-(2,4-difluorobenzyl)-3-(4-fluoro-2-methoxyphenyl)cinnolin-4(1*H*)-one;
- 3-(2,3-dichlorophenyl)-1-(2,4-difluorobenzyl)cinnolin-4(1*H*)-one;
- 1-(2,4-difluorobenzyl)-3-(5-fluoro-2-methoxyphenyl)cinnolin-4(1*H*)-one;
- 1-(2,4-difluorobenzyl)-3-(2-methoxyphenyl)cinnolin-4(1*H*)-one;
- 3-(3-bromo-2-fluorophenyl)-1-(2,4-difluorobenzyl)cinnolin-4(1*H*)-one;
- 1-(2,4-difluorobenzyl)-3-(2-fluoro-3-methylphenyl)cinnolin-4(1*H*)-one;
- 1-(2,4-difluorobenzyl)-3-[2-(trifluoromethyl)phenyl]cinnolin-4(1*H*)-one;
- 1-(2,4-difluorobenzyl)-3-[2-(trifluoromethoxy)phenyl]cinnolin-4(1*H*)-one;
- 1-(2,4-difluorobenzyl)-3-[3-(trifluoromethoxy)phenyl]cinnolin-4(1*H*)-one;
- 1-(2-chloro-4-fluorobenzyl)-3-[2-fluoro-3-(trifluoromethyl)phenyl]cinnolin-4(1*H*)-one;
- 1-(4-chloro-2-fluorobenzyl)-3-(5-fluoro-2-methoxyphenyl)cinnolin-4(1*H*)-one;
- 3-(3-chloro-2-fluorophenyl)-1-(2,4-difluorobenzyl)cinnolin-4(1*H*)-one;
- 3-fluoro-4-({4-oxo-3-[2-(trifluoromethyl)phenyl]cinnolin-1(4*H*)-yl}methyl)benzonitrile;
- 1-(2,4-difluorobenzyl)-3-(4-methylphenyl)cinnolin-4(1*H*)-one;
- 1-(2,4-difluorobenzyl)-3-[3-(trifluoromethyl)phenyl]cinnolin-4(1*H*)-one;
- 1-[(5-chlorothien-2-yl)methyl]-3-[2-fluoro-3-(trifluoromethyl)phenyl]cinnolin-4(1*H*)-one;
- 1-(2,4-difluorobenzyl)-3-[2-(trifluoromethyl)pyridin-3-yl]cinnolin-4(1*H*)-one;
- 3-(1-benzothien-7-yl)-1-(2,4-difluorobenzyl)cinnolin-4(1*H*)-one;
- 1-benzyl-3-(3-isopropylphenyl)cinnolin-4(1*H*)-one;
- 1-(2,4-difluorobenzyl)-3-(5-isopropyl-2-methoxyphenyl)cinnolin-4(1*H*)-one;
- 1-(2,4-difluorobenzyl)-3-(1-naphthyl)cinnolin-4(1*H*)-one;
- 1-(2,4-difluorobenzyl)-3-[2-(trifluoromethyl)pyridin-3-yl]cinnolin-4(1*H*)-one;
- 1-(2,4-difluorobenzyl)-3-[2-fluoro-5-(trifluoromethoxy)phenyl]cinnolin-4(1*H*)-one;
- 1-(2,4-difluorobenzyl)-3-(4-methoxyphenyl)cinnolin-4(1*H*)-one;
- 3-(2,2-difluoro-1,3-benzodioxol-4-yl)-1-(2,4-difluorobenzyl)cinnolin-4(1*H*)-one;
- 3-(4-chlorophenyl)-1-(2,4-difluorobenzyl)cinnolin-4(1*H*)-one;
- 1-[(5-chlorothien-2-yl)methyl]-3-(2,3-dichlorophenyl)cinnolin-4(1*H*)-one;
- 1-(4-chloro-2-fluorobenzyl)-3-[3-(trifluoromethyl)phenyl]cinnolin-4(1*H*)-one;
- 1-(4-fluorobenzyl)-3-(5-fluoro-2-methoxyphenyl)cinnolin-4(1*H*)-one;
- 1-(2-fluoro-4-methylbenzyl)-3-[2-fluoro-3-(trifluoromethyl)phenyl]cinnolin-4(1*H*)-one;
- 1-(2-fluorobenzyl)-3-(5-fluoro-2-methoxyphenyl)cinnolin-4(1*H*)-one;
- 1-(2,4-difluorobenzyl)-3-(2-fluoro-4-methylphenyl)cinnolin-4(1*H*)-one;
- 1-(4-fluorobenzyl)-3-[3-(trifluoromethyl)phenyl]cinnolin-4(1*H*)-one;
- 1-(2,4-difluorobenzyl)-3-(3-fluorophenyl)cinnolin-4(1*H*)-one;
- 3-(5-fluoro-2-methoxyphenyl)-1-[2-fluoro-4-(trifluoromethyl)benzyl]cinnolin-4(1*H*)-one;
- 1-(4-fluorobenzyl)-3-[2-fluoro-3-(trifluoromethyl)phenyl]cinnolin-4(1*H*)-one;
- 3-(5-fluoro-2-methoxyphenyl)-1-(2-fluoro-4-methylbenzyl)cinnolin-4(1*H*)-one;
- 1-(2-chloro-4-fluorobenzyl)-3-(5-fluoro-2-methoxyphenyl)cinnolin-4(1*H*)-one;
- 1-(2,4-difluorobenzyl)-3-(2,5-difluorophenyl)cinnolin-4(1*H*)-one;
- 1-(2,4-difluorobenzyl)-3-[3-fluoro-5-(trifluoromethyl)phenyl]cinnolin-4(1*H*)-one;
- 1-(2,4-difluorobenzyl)-3-(2-methylphenyl)cinnolin-4(1*H*)-one;
- 1-(2,4-difluorobenzyl)-3-(2-naphthyl)cinnolin-4(1*H*)-one;
- 1-(2,4-difluorobenzyl)-3-(2,3-difluorophenyl)cinnolin-4(1*H*)-one;
- 1-[2-fluoro-4-(trifluoromethyl)benzyl]-3-[2-fluoro-3-(trifluoromethyl)phenyl]cinnolin-4(1*H*)-one;
- 3-[3-chloro-5-(trifluoromethyl)phenyl]-1-(2,4-difluorobenzyl)cinnolin-4(1*H*)-one;
- 3-(3,5-dichlorophenyl)-1-(2,4-difluorobenzyl)cinnolin-4(1*H*)-one;
- 1-(2,4-difluorobenzyl)-3-(2-fluorophenyl)cinnolin-4(1*H*)-one;
- 1-(2,4-dichlorobenzyl)-3-(5-fluoro-2-methoxyphenyl)cinnolin-4(1*H*)-one;
- 3-[2-fluoro-3-(trifluoromethyl)phenyl]-1-{[5-(trifluoromethyl)pyridin-2-yl]methyl}cinnolin-4(1*H*)-one;
- 3-(5-(tert-butyl)-2-methoxyphenyl)-1-(2,4-difluorobenzyl)cinnolin-4(1*H*)-one;
- 1-[2-fluoro-4-(trifluoromethyl)benzyl]-3-[3-(trifluoromethyl)phenyl]cinnolin-4(1*H*)-one;
- 3-[4-chloro-2-(trifluoromethyl)phenyl]-1-(2,4-difluorobenzyl)cinnolin-4(1*H*)-one;
- 1-(2,4-difluorobenzyl)-3-(5-fluoro-2-hydroxyphenyl)cinnolin-4(1*H*)-one;
- 1-(2,4-difluorobenzyl)-3-phenylcinnolin-4(1*H*)-one;
- 1-(2,4-dichlorobenzyl)-3-[3-(trifluoromethyl)phenyl]cinnolin-4(1*H*)-one;
- 1-(2-chloro-4-fluorobenzyl)-3-[3-(trifluoromethyl)phenyl]cinnolin-4(1*H*)-one;
- 1-(2-fluoro-4-methylbenzyl)-3-[3-(trifluoromethyl)phenyl]cinnolin-4(1*H*)-one;
- 3-(1-benzothien-2-yl)-1-(4-chlorobenzyl)cinnolin-4(1*H*)-one;
- 1-(2-fluorobenzyl)-3-[2-fluoro-3-(trifluoromethyl)phenyl]cinnolin-4(1*H*)-one;
- 1-[(5-chlorothien-2-yl)methyl]-3-(5-fluoro-2-methoxyphenyl)cinnolin-4(1*H*)-one;
- 1-(2,4-difluorobenzyl)-3-[4-(trifluoromethoxy)phenyl]cinnolin-4(1*H*)-one;
- 1-(2,4-difluorobenzyl)-3-(6-methoxypyridin-3-yl)cinnolin-4(1*H*)-one;
- 3-(2,3-dichlorophenyl)-1-{[5-(trifluoromethyl)pyridin-2-yl]methyl}cinnolin-4(1*H*)-one;
- 7-chloro-1-(2,4-difluorobenzyl)-3-[2-fluoro-3-(trifluoromethyl)phenyl]cinnolin-4(1*H*)-one;
- 1-(2,4-difluorobenzyl)-3-[3-fluoro-2-(trifluoromethyl)phenyl]-7-methoxycinnolin-4(1*H*)-one;
- 7-chloro-3-[2-chloro-3-(trifluoromethyl)phenyl]-1-(2,4-difluorobenzyl)cinnolin-4(1*H*)-one;
- 1-(2,4-difluorobenzyl)-7-methoxy-3-[3-(trifluoromethoxy)phenyl]cinnolin-4(1*H*)-one;
- 4-({3-[2-chloro-3-(trifluoromethyl)phenyl]-4-oxocinnolin-1(4*H*)-yl}methyl)-3-fluorobenzonitrile;
- 7-chloro-1-(2,4-difluorobenzyl)-3-[3-fluoro-2-(trifluoromethyl)phenyl]cinnolin-4(1*H*)-one;
- 7-chloro-3-(2,3-dichlorophenyl)-1-(2,4-difluorobenzyl)cinnolin-4(1*H*)-one;
- 7-chloro-1-(2,4-difluorobenzyl)-3-[3-(trifluoromethoxy)phenyl]cinnolin-4(1*H*)-one;
- 7-chloro-1-(2,4-difluorobenzyl)-3-[2-(methylsulphonyl)phenyl]cinnolin-4(1*H*)-one;
- 1-[(5-chloropyridin-2-yl)methyl]-3-[2-fluoro-3-(trifluoromethyl)phenyl]cinnolin-4(1*H*)-one;
- 6-chloro-3-[2-chloro-3-(trifluoromethyl)phenyl]-1-(2,4-difluorobenzyl)cinnolin-4(1*H*)-one;
- 6-chloro-1-(2,4-difluorobenzyl)-3-[2-fluoro-3-(trifluoromethyl)phenyl]cinnolin-4(1*H*)-one;
- 1-(2,4-difluorobenzyl)-3-[2-fluoro-3-(trifluoromethyl)phenyl]-6-methoxycinnolin-4(1*H*)-one;
- 6-chloro-1-[(3,5-difluoropyridin-2-yl)methyl]-3-[2-fluoro-3-(trifluoromethyl)phenyl]cinnolin-4(1*H*)-one; in the form of the base or of an addition salt with an acid.

5. Process for the preparation of the compounds of formula (I) according to any one of Claims 1 to 4, **characterized in that**:
a compound of formula: 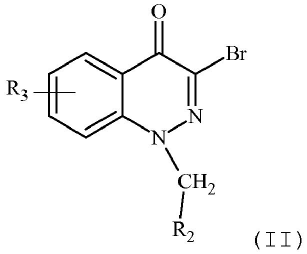 in which R₂ and R₃ are as defined for a compound of formula (I) in Claim 1, is reacted in the presence of a base with a compound of formula:
R₁-B(OH)₂ (III)
in which R₁ is as defined for a compound of formula (I) in Claim 1.

6. Medicament, **characterized in that** it comprises a compound of formula (I) according to any one of Claims 1 to 4 or a pharmaceutically acceptable salt of the compound of formula (I).

7. Pharmaceutical composition, **characterized in that** it comprises a compound of formula (I) according to any one of Claims 1 to 4, a pharmaceutically acceptable salt of this compound and at least one pharmaceutically acceptable excipient.

8. Use of a compound of formula (I) according to any one of Claims 1 to 4 in the preparation of a medicament intended to treat and prevent any pathology in which CB₂ cannabinoid receptors are involved.

9. Use according to Claim 8 for the treatment and prevention of pain, inflammatory diseases, autoimmune diseases, allergic diseases, infectious diseases, neurodegenerative diseases, cardiovascular diseases, cancers, gastrointestinal diseases, obesity, type II diabetes, insulin resistance and adipose tissue inflammation.
